# FASCICULE DE BREVET EUROPEEN

(11) **EP 0 706 392 B1**
(45) Date de publication et mention de la délivrance du brevet: **03.11.1999**
(21) Numéro de dépôt: 94920995.1
(22) Date de dépôt: 29.06.1994
(51) Int. Cl.: A61K 31/34

(54) **UTILISATION DES DERIVES DU ROSMANOL POUR LA FABRICATION DES MEDICAMENTS ANTI-RADICALAIRES ET/OU ANTILIPOPEROXYDANTES ET/OU HEPATOTROPES**
VERWENDUNG VON ROSMANOLDERIVATEN ZUR HERSTELLUNG VON ANTIRADIKALISCHEN UND/ODER ANTILIPOPEROXIDATIVEN UND/ODER HEPATOTROPEN ARZNEISTOFFEN
USE OF ROSMANOL DERIVATIVES FOR PRODUCING ANTIRADICAL AND/OR ANTILIPOPEROXIDATIVE AND/OR HEPATOTROPIC DRUGS

(30) Priorité: 30.06.1993 FR 9307983
(43) Date de publication de la demande: 17.04.1996
(73) Titulaire: LABORATOIRES DOLISOS, 75003 Paris (FR)
(72) Inventeur: JOYEUX, Michel, F-54110 Varangeville (FR); MORTIER, François, F-54220 Malzeville (FR)
(74) Mandataire: Ahner, Francis
(86) Numéro de dépôt international: FR9400793
(87) Numéro de publication internationale: WO9501174

(56) Documents cités:
- DATABASE WPI Week 8534, Derwent Publications Ltd., London, GB; AN 85-207789 & JP,A,60 132 987 (RIKEN VITAMIN CO KK) 16 Juillet 1985
- DATABASE WPI Week 8430, Derwent Publications Ltd., London, GB; AN 84-185215 & JP,A,59 103 665 (LION CORP) 15 Juin 1984
- PLANTA MED., vol.58, no.7, 1992 page A632 PUKL, M. ET AL 'INHIBITORY EFFECT OF CARNOSOLIC ACID ON HIV-1 PROTEASE'
- AGRICULTURAL AND BIOLOGICAL CHEMISTRY, vol.48, no.8, 1984 pages 2081 - 2085 NAKATANI, N. ET AL 'TWO ANTIOXIDATIVE DITERPENES FROM ROSEMARY (ROSMARINUS OFFICINALIS L.) AND A REVISED STRUCTURE FOR ROSMANOL'
- AGRICULTURAL AND BIOLOGICAL CHEMISTRY, vol.47, no.3, 1983 pages 521 - 528 INATANI, R. ET AL 'ANTIOXIDATIVE EFFECT OF THE CONSTITUENTS OF ROSEMARY (ROSMARINUS OFFICINALIS L.) AND THEIR DERIVATIVES'
- Z. LEBENSM. UNTERS. FORSCH., vol.195, no.2, 1992 pages 95 - 98 SCHWARZ, K. ET AL 'ANTIOXIDATIVE CONSTITUENTS OF ROSMARINUS OFFICINALIS AND SALVIA OFFICINALIS. I. DETERMINATION OF PHENOLIC DITERPENES WITH ANTIOXIDATIVE ACTIVITY AMONGST TOCOCHROMANOLS USING HPLC'

## Description

L'invention concerne des compositions pharmaceutiques antiradicalaires et/ou antilipoperoxydantes et/ou hépatotropes.

Dans nos régions, les affections hépatiques sont dominées par deux grands groupes étiologiques:
- les pathologies hépatiques de nature infectieuse,
- les pathologies hépatiques de nature toxique.

Leurs expressions cliniques, biologiques ou encore histologiques sont d'ailleurs souvent proches voire identiques, ce qui n'est pas sans poser des problèmes de diagnostic différentiel.

Ces distinctions étiologiques ne se retrouvent pas dans la plupart des pharmacopées traditionnelles à visée hépatique où les usages des extraits végétaux sont justifiés en termes peu précis comme "indiqué dans les affections hépatiques", ce qui peut recouvrir des pathologies extrêmement variées.

Il existe par ailleurs un point de discordance entre les Médecines dites traditionnelles et la Médecine dite moderne. Les premières, malgré leur manque de rigueur sémiologique, proposent une pléthore de remèdes. De très nombreux extraits végétaux sont utilisés de manière empirique en morothérapie ou en association par les tradipraticiens. Plusieurs centaines de plantes sont ainsi "indiquées dans les affections hépatiques". La seconde, malgré des connaissances souvent très précises des mécanismes morbides et l'existence de classifications étiologiques parfois très subtiles, ne propose qu'une gamme thérapeutique modeste.

Les indications thérapeutiques de *Rosmarinus officinalis* sont très nombreuses et utilisent les différentes parties de la plante. On a relevé notamment des propriétés diurétiques, antiseptiques, antirhumatismales, cicatrisantes, astringentes, emménagogues. *Rosmarinus officinalis* a surtout une place privilégiée dans le domaine des affections hépatobiliaires. *Rosmarinus officinalis* est constitué de nombreux principes actifs. Son action sur le foie (cholagogue et cholestérique) est liée essentiellement à la présence de l'acide rosmarinique.

Le rosmanol est un composé du groupe des lactones diterpéniques et qui a été isolé à partir de *Rosmarinus officinalis* (Inatani et al; 1982: "Structure of the new antioxydative phenolic diterpene isolated from Rosmary", Agric. Biol. Chem., 46, 1661 et suivantes). Le rosmanol est connu comme possédant une activité antioxydante (dans divers tests non biologiques), pouvant donner lieu à des applications en tant qu'antioxydants alimentaires (test de l' oxydation des "tranches de lard").

Le rosmanol a également été isolé à partir de Salvia *canariensis* et sa structure a été parfaitement déterminée par analyse chimique et par diffraction aux rayons X. (Fraga et al., 1985: "A revised structure for the diterpene rosmanol", Phytochemistry, Vol. 24, N° 8, 1853-1854).

Le rosmanol ainsi que le 7-éthoxyrosmanol ont été également extraits de *Rosmarinus officinalis* (Studies on Medicinal Plants in Paraguay; Studies on "Romero"; part 1; T. Hayashi, Planta medica 1987; vol. 53, P 394).

Cet article indique que le rosmanol présente une activité inhibitrice contre l'uréase, mais aucune corrélation n'est établie de façon certaine et non ambigüe avec la mise en oeuvre d'une méthode de traitement thérapeutique.

Par ailleurs, à ce jour, on n'avait fait aucun lien entre les propriétés pharmacologiques, notamment hépatoprotectrices de *Rosmarinus officinalis* et la substance active responsable de ces propriétés.

L'invention a permis de mettre en évidence le composé chimique déterminé porteur de l'activité antiradicalaire, antilipoperoxydante et hépatotrope de *Rosmarinus officinalis*.

L'invention a pour objet de nouvelles compositions pharmaceutiques, dont la substance active susceptible de provenir de plantes, et notamment de *Rosmarinus officinalis*, est totalement identifiée et peut donc être dosée.

L'invention a pour objet une composition pharmaceutique caractérisée en ce qu'elle contient, comme substance active l'un au moins des composés de formule I: 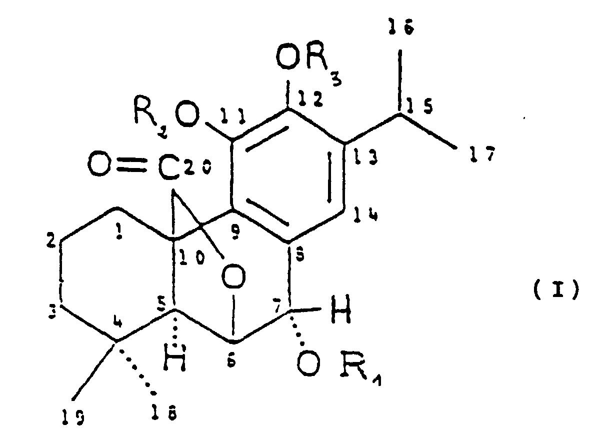 dans laquelle R₁, R₂, R₃ identiques ou différents représentent H, CH₃ ou C₂H₅, éventuellement en association avec un sel pharmaceutiquement acceptable.

L'invention couvre également les molécules de formule (I) dans lesquelles l'une au moins des positions substituables (notamment les positions 15 et/ou 16, et/ou 17, et/ou 18, et/ou 19), peut être substituée par des substituants tels que les molécules gardent leurs propriétés antiradicalaires, et/ou antilipoperoxydantes et/ou hépatotropes, ces molécules étant des équivalents des composés de formule I.

Les composés de formule I ont des activités antiradicalaires et antilipoperoxydantes.

Dans *Rosmarinus officinalis*, ces composés ont également une action hépatoprotectrice, résultant de l'association avec d'autres principes actifs du romarin.

Selon un mode de réalisation avantageux de l'invention, les compositions pharmaceutiques contiennent, comme substance active le composé de formule II:

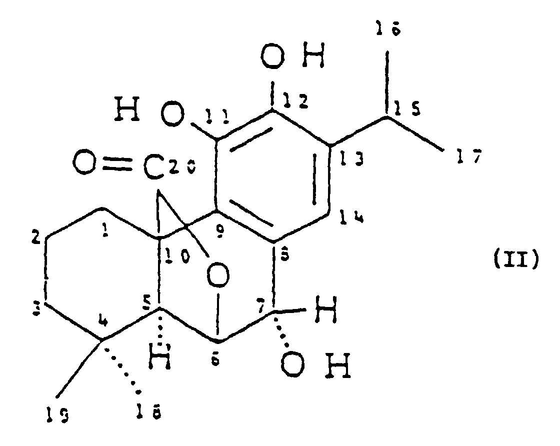

Ce composé est le rosmanol.

Un autre groupe de compositions pharmaceutiques avantageux selon l'invention contient, comme substance active, le composé de formule III:

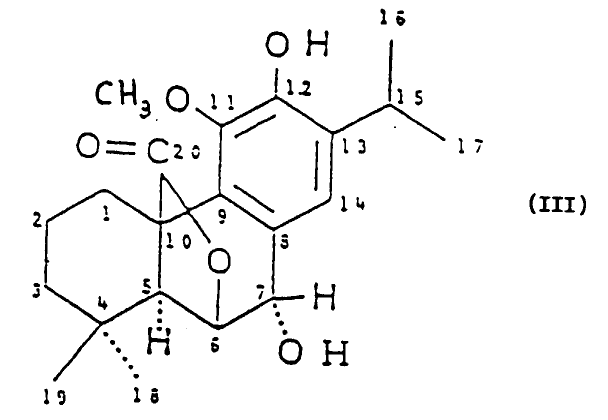

Ce composé est le 11-méthoxyrosmanol.

Un autre groupe de compositions pharmaceutiques avantageux selon l'invention contient, comme substance active, le composé de formule IV:

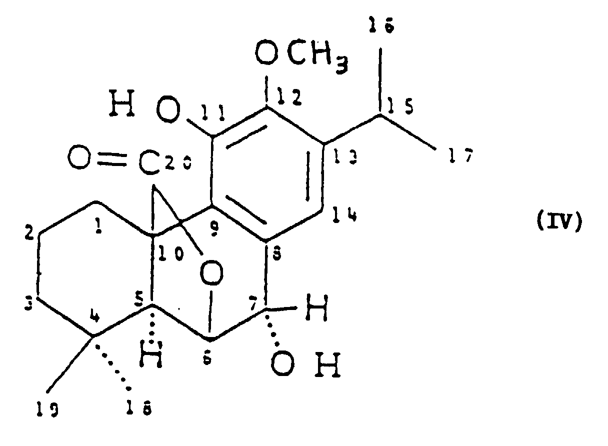

Ce composé est le 12-méthoxyrosmanol.

Un autre groupe de compositions pharmaceutiques avantageux selon l'invention contient, comme substance active, le composé de formule V:

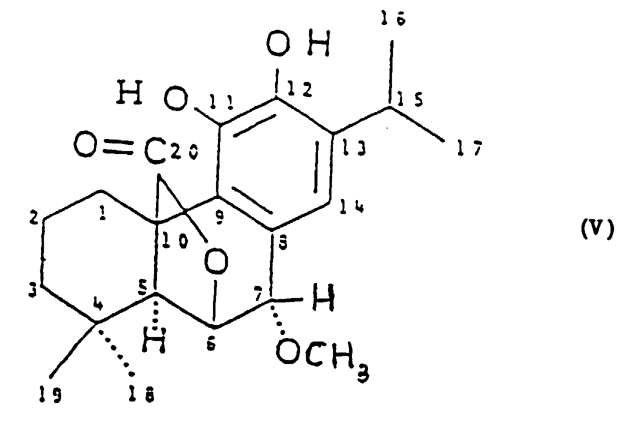

Ce composé est le 7-méthoxyrosmanol.

Un autre groupe avantageux de compositions pharmaceutiques selon l'invention contient, comme substance active un composé de formule VI:

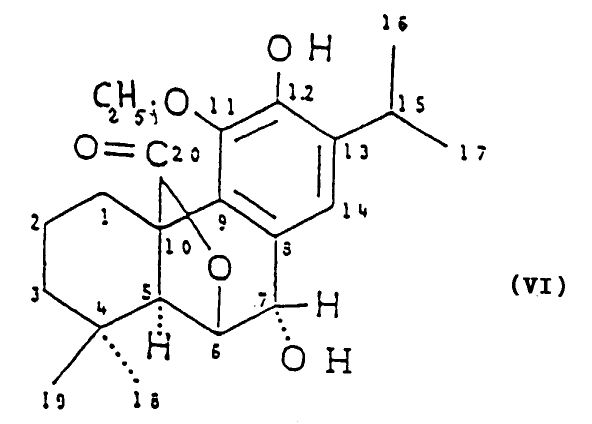

Ce composé est le 11-ethoxyrosmanol.

Un autre groupe avantageux de compositions pharmaceutiques selon l'invention contient, comme substance active un composé de formule VII:

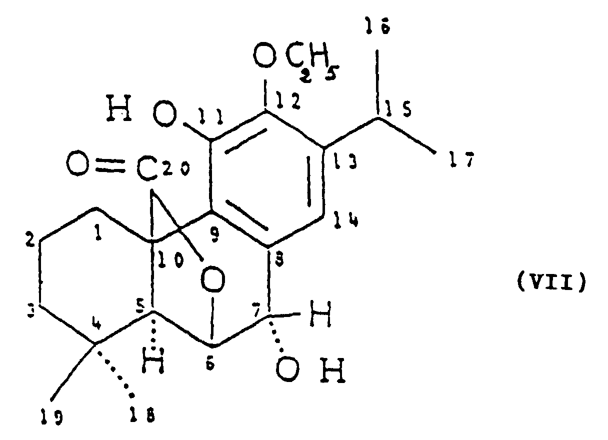

Ce composé est le 12-éthoxyrosmanol.

Un autre groupe avantageux de compositions pharmaceutiques selon l'invention contient, comme substance active un composé de formule VIII:

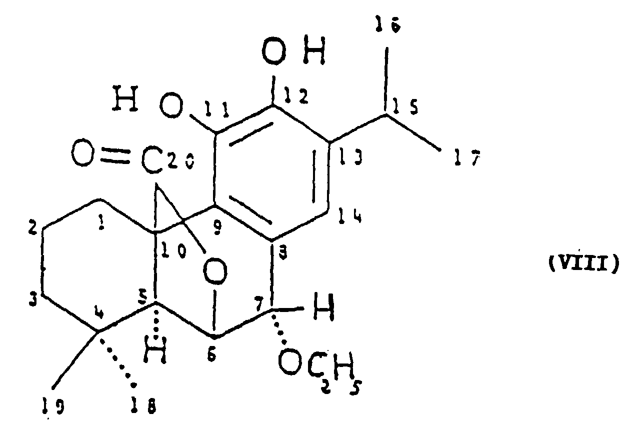

Ce composé est le 7-éthoxyrosmanol.

L'invention a pour objet des compositions pharmaceutiques ayant des propriétés antiradicalaires et/ou antilipoperoxydantes et/ou hépatotropes, contenant, comme substance active, l'un au moins des composés de formule I.

Selon un autre mode de réalisation avantageux, l'invention a pour objet des compositions pharmaceutiques ayant des propriétés antiradicalaires et/ou antilipoperoxydantes et/ou hépatotropes, contenant comme substance active l'un au moins des composés de formule II, III, IV, V, VI, VII ou VIII.

Selon un autre mode de réalisation avantageux, l'invention a pour objet des compositions pharmaceutiques ayant des propriétés antiradicalaires et/ou antilipoperoxydantes et/ou hépatotropes, contenant comme substance active l'un au moins des composés de formule II, V, ou VIII.

L'invention a également pour objet une composition pharmaceutique, caractérisée en ce qu'elle contient d'environ 0,1mg/kg à environ 100 mg/kg de substance active constituée de l'un au moins des composés de formule I, et notamment d'environ 1 mg/kg à environ 10 mg/kg de substance active.

L'expression "mg/kg" correspond au mg de substance active par kilogramme de poids corporel.

L'invention a également pour objet une composition pharmaceutique, caractérisée en ce qu'elle contient d'environ 0,1 mg/kg à environ 100 mg/kg. de substance active constituée de l'un au moins des composés de formule II, III, IV, V, VI, VII ou VIII, et notamment d'environ 1 mg/kg à environ 10 mg/kg de substance active.

L'invention a également pour objet une composition pharmaceutique, caractérisée en ce qu'elle contient d'environ 0,1 mg/kg à environ 100 mg/kg de substance active constituée de l'un au moins des composés de formule II, V,ou VIII, et notamment d'environ 1 mg/kg à environ 10 mg/kg de substance active.

De façon avantageuse, la dose journalière de substance active est d'environ 2 à environ 30 mg/kg et notamment d'environ 3 à environ 20 mg/kg.

Les compositions pharmaceutiques de l'invention, sont caractérisées en ce qu'elles se présentent sous une forme appropriée à l'administration par voie orale, parentérale, rectale ou topique.

En particulier, on peut citer les comprimés, dragées, capsules, gélules, poudres, granulés, suspensions ou sirops pour l'administration orale, les suppositoires pour la voie rectale, les solutions stériles ou stérilisables pour la voie parentérale et les collyres ou crèmes pour la voie topique.

Les composés de l'invention de formule II, III, IV, V, VI, VII ou VIII peuvent être extraits de *Rosmarinus officinalis*.

Le rosmanol peut être préparé selon le procédé décrit par exemple par T. Hayashi et al., dans "Studies on Medicinal Plants in Paraguay: Studies on Romero"; Part 1, Planta medica 1987, vol. 53, p. 394.

Le 7-méthoxyrosmanol peut être préparé selon le procédé décrit par Munehisa Arisawa et ai., dans "Chemical and Pharmaceutical Studies on Medicinal Plants in Paraguay: Studies on "Romero", Part 2", Journal of Natural Products, Vol. 50, N° 6, pp. 1164-1166, Nov. Dec. 1987.

Le 7-éthoxyrosmanol peut être préparé selon le procédé décrit par T. Hayashi et al., dans "Studies on Medicinal Plants in Paraguay: Studies on Romero"; Part 1, Planta medica 1987, vol. 53, p. 394.

Les composés de formule III, IV ou V peuvent être également obtenus à partir du composé de formule II, soumis à une méthylation, dans les conditions appropriées selon, par exemple, des techniques classiquement utilisées pour le dosage des composés diterpéniques ou triterpéniques en chromatographie gazeuse.

Les composés de formule VI, VII ou VIII peuvent être obtenus à partir du composé de formule II, soumis à une éthylation dans des conditions appropriées.

L'invention a pour objet l'utilisation de l'un au moins des composés de formule I et notammment de formule II, III, IV, V, VI, VII ou VIII ci-dessus définis, pour la préparation d'un médicament destiné au traitement de pathologies liées à des mécanismes de formation de radicaux libres, ou au traitement de pathologies liées à un mécanisme de lipoperoxydation, ou au traitement ou à la protection des cellules hépatiques.

L'invention a pour objet l'utilisation du composé de formule II ci-dessus défini, pour la préparation d'un médicament destiné au traitement de pathologies liées à des mécanismes de formation de radicaux libres, ou au traitement de pathologies liées à un mécanisme de lipoperoxydation, ou au traitement ou à la protection des cellules hépatiques.

L'invention a pour objet l'utilisation du composé de formule V ci-dessus défini, pour la préparation d'un médicament destiné au traitement de pathologies liées à des mécanismes de formation de radicaux libres, ou au traitement de pathologies liées à un mécanisme de lipoperoxydation, ou au traitement ou à la protection des cellules hépatiques.

L'invention a pour objet l'utilisation du composé de formule VIII ci-dessus défini, pour la préparation d'un médicament destiné au traitement de pathologies liées à des mécanismes de formation de radicaux libres, ou au traitement de pathologies liées à un mécanisme de lipoperoxydation, ou au traitement ou à la protection des cellules hépatiques.

Selon un mode de réalisation avantageux, l'invention concerne l'utilisation des composés de formule I, pour la préparation d'un médicament destiné aux pathologies liées à un mécanisme de lipoperoxydation. C'est le cas notamment du traitement des problèmes de revascularisation, par exemple, dans le cadre des accidents vasculaires cérébraux ou dans le cadre de l' infarctus; c'est également le cas du traitement de l'artérite.

### EXAMPLE:

### EXTRACTION DU ROSMANOL ET DU METHYL-ROSMANOL ET DETERMINATION DE LEUR ACTIVITE PHARMACOLOGIQUE

### A) SELECTION DE ROSMARINUS OFFICINALIS

Au cours de ce travail, 26 espèces végétales ont été étudiées. Pour chacune d'elles, lorsque cela a été possible, une rapide identification chromatographique est réalisée sur couche mince.

Pour chaque extrait végétal, l'activité antiradicalaire est recherchée à l'aide du test de décoloration du radical diphényl picryl hydrazyl comme précisé ci-après. Les extraits sont classés en deux catégories selon que la coloration obtenue est inférieure ou supérieure à 50% de celle du blanc témoin. L'étude de l'activité antilipoperoxydante et antinécrotique est réalisée à l'aide du modèle d'hépatocytes isolés, intoxiqués au tert-butyl hydroperoxyde dont la méthodologie a été précédemment décrite. Les doses expérimentées sont toujours en mg de plantes sèches.

A coté d'espèces végétales traditionnellement utilisées pour leurs vertus hépatotropes, d'autres plantes, dont les indications traditionnelles sont étrangères à la sphère hépatobiliaire, sont également testées afin d'approcher la pertinence des modèles pharmacologiques retenus.

Les espèces végétales, traditionnellement hépatotropes, étudiées au cours de ce travail sont au nombre de 17, il s'agit de:
- *Rosmarinus officinalis*: *Eupatorium cannabinum*
- *Cuscuta americana*: *Euphorbia hirta*
- *Curcuma domestica*: *Juniperus communis*
- *Terminalia catappa*: *Crepis rueppellii*
- *Tamarindus indica*: *Pulicaria orientalis*
- *Kalanchoe crenata*: *Bidens pilosa*
- *Staudia gabonensis*: *Carthamus tinctorium*
- *Cochlospermum tinctorium*: *Peumus boldus*
- *Cochlospermum planchonii*:

Les autres espèces végétales sont au nombre de 9, il s'agit de:
- *Momordica charantia*: *Eschscholzia californica*
- *Justicia pectoralis*: *Vaccinium myrtillus*
- *Annona muricata*: *Annona reticulata*
- *Eupatorium xanthoxyloïdes*: *Capparis spinosa*

Pour ce faire, deux modèles d'étude ont été adaptés à l'utilisation des espèces végétales ci-dessus:
- un test non biologique, le test de décoloration du radical diphényl picryl hydrazyl (DPPH),
- un test biologique utilisant des hépatocytes isolés, maintenus en suspension.

### Test du DPPH

Ce modèle, non biologique, permet d'approcher l'activité antiradicalaire des substances testées. Parmi toutes les techniques pouvant être utilisées pour cette étude, la décoloration du radical diphénylpicryl hydrazyl (Deby et coll., 1970 Relation entre les acides gras essentiels et le taux des antioxydants tissulaires chez la souris. C.R. Soc. Biol., 164, 2675-2681), nous a semblé être le moyen le plus adapté dans le cadre d'un criblage du fait de sa rapidité de sa facilité de mise en oeuvre et de son coût modique.

Le radical diphényl picryl hydrazyl est un radical libre, stable sous certaines conditions qui présente en solution, une intense coloration violette de maximum d'absorption à 517 nm. Cette coloration disparaît lors de la saturation des couches électroniques, c'est à dire lors d'un passage vers une forme non radicalaire. De ce fait, la diminution de la densité optique de la solution rend compte du pouvoir piégeur des radicaux libres de la substance ajoutée. Le degré d'inhibition est exprimé par rapport à un blanc témoin traité dans les mêmes conditions.

Le modèle appliqué jusqu'à présent sur des molécules pures a dû être adapté à l'étude d'extraits végétaux par la définition des concentrations d'étude. On a ainsi pu établir que des solutions d'extraits végétaux à 0.25 et 0.125g/l permettent, selon les extraits considérés, de couvrir toute la gamme de décoloration de 0 à 100%. Ne possédant aucun recul bibliographique, le critère d'efficacité a, dans un premier temps été arbitrairement défini à 50% de décoloration, la valeur prédictive des résultats obtenus sur ce test étant secondairement affinée par la confrontation avec les résultats du test biologique sur hépatocytes isolés.

### Test sur hépatocytes isolés

LA second modèle d'étude est un modèle biologique, ayant pour support des hépatocytes de rat fraîchement isolés et maintenus en suspension. Il permet l'approche du pouvoir antilipoperoxydant des extraits végétaux par le dosage d'un des produits terminaux de la peroxydation lipidique, la malonaldéhyde (MAD) ainsi que celle de leur pouvoir antinécrotique par la quantification du relargage extracellulaire de la lactico déshydrogénase (LDH) et de l'aspartate amino-transférase (ASAT), marqueurs biologiques classiques des phénomènes de cytolyse.

De nombreux travaux ont montré que le modèle d'hépatocytes isolés en suspension et maintenus en survie sur de courtes périodes, inférieures à quelques heures, permet de conserver une part essentielle de l'intégrité et de la qualité de la cellule hépatique, autorisant son utilisation dans l'étude de divers mécanismes biochimiques ou toxicologiques (Klaasen et Stacey, 1982, 147-180, "Toxicology of the liver" PLAA G.L. & Hewitt W.R., Raven Press, New York; Krack et coll., 1983: "Metabolic competence of isolated hepatocytes in suspension. A new tool for in vitro toxicological evaluation", 391-398, Isolation, characterization and use of hepatocytes, Harris R.A. & Cornell N.W. (eds) ; Arvis, 1989: "Utilisation des hépatocytes en culture pour le criblage toxico-pharmacologique de substances naturelles hépatotropes", 236 p., Doctorat de Sciences de l'Université de Metz, Metz).

### Matériels et méthodes

### I) Choix du toxique

### Tert-butylhydroperoxyde (tBH) et hépatocytes

LA tert-butylhydroperoxyde [(CH₃)-₃COOH] est un hydroperoxyde organique, de poids moléculaire égal à 90.1, pouvant être utilisé pour l'étude de mécanismes oxydatifs. Il entraîne, au niveau hépatocytaire, une peroxydation lipidique, la formation de liaisons covalentes sur les macromolécules cellulaires ainsi qu'une perturbation de l'homéostasie calcique.

### II) Préparation des hépatocytes en suspension

### 1. Isolement des hépatocytes

### 1.1 Animaux

Rats mâles Sprague - Dawley OFA (Iffa Credo, l'Arbresle), 200 à 250 grammes, nourris (croquettes M25C Extralabo) et abreuvés *ad libitum*. Les animaux sont stabulés dans des cages en macrolon et maintenus dans une animalerie cyclée (12 heures d'éclairage/ 12 heures d'obscurité) et thermostatée (21 ± 1°C).

### 1.2 Matériels

Les produits utilisés ont été les suivants: collagénase (Sigma), milieu minimum de Eagle (Flow Laboratory), tert-butyl hydroperoxyde (Sigma), hépes (Sigma), acide trichloroacétique (Prolabo), acide 2-thiobarbiturique (Prolabo), diméthyl-sulfoxide (Prolabo), kit LDH Opt. (Boehringer), kit GOT Opt. (Boehringer).

### 1.3 Technique d'isolement des hépatocytes

L'animal est anesthésié au pentobarbital sodique (Clin Midy) à la dose de 60 mg/kg par voie IP puis hépariné à raison de 1 ml/kl (héparine 50 Fournier 5000 Ul) selon la même voie d'injection et placé en décubitus dorsal dans une enceinte thermostatée à 37 ± 1 °C.

Après laparotomie et excision de la paroi abdominale, un cathéter (Critikon 18G) est mis en place dans la veine porte hépatique. La veine cave inférieure est sectionnée sous les veines rénales et le foie est perfusé à un débit de 30 ml/minute au moyen d'une pompe péristaltique (Masterflex) par un tampon phosphate, pH 7,4. Durant cette phase de lavage, après thoracotomie, un second cathéter est poussé par voie auriculaire dans la veine cave supérieure permettant la mise en place du dispositif assurant la recirculation du milieu de perfusion. Lorsque le lavage est jugé correct, le foie est perfusé à l'aide d'une solution de collagénase 0,05% p/v et de CaCl₂ 5mM à un débit de 20ml/mn. Lorsque l'action dissociatrice de l'enzyme est jugée suffisante, le foie est excisé puis placé dans une boîte de Pétri. La capsule de Glisson est délicatement retirée et les cellules sont mises en suspension par agitation douce. Le tissu conjonctif et le système artério-veineux ainsi que les amas cellulaires non dissociés sont écartés par filtration sur tamis de soie (68µ).

Deux décantations-lavages sont alors effectuées, le culot étant remis en suspension dans un milieu minimum d'Eagle (MEM) tamponné par 20mM d'Hépes. Le comptage est réalisé sur une cellule de Malassez. La viabilité est appréciée par le test d'exclusion du bleu Trypan. Le rendement est de l'ordre de 3 à 5 10⁸ cellules et la viabilité initiale doit être supérieure à 90%.

### 1.4 Protocole expérimental

La concentration de la suspension d'hépatocytes est ajustée à 1 million de cellules par ml de milieu nutritif. Des fractions aliquotes de 4 ml de suspension sont réparties dans des Erlenmeyer de 25 ml recouverts de parafilm, maintenus à 37°C au bain-marie sous agitation (60 oscillations/mn); chaque échantillon est gazé individuellement par un mélange O₂/CO₂ (95%/5%). Les différents toxiques utilisés sont ajoutés au milieu de culture directement ou après solubilisation dans du diméthyl sulfoxide (DMSO). La concentration finale en DMSO est de 0,5% v/v. Les conditions d'utilisation propres à chaque toxique sont décrites dans les paragraphes les concernant. Les extraits végétaux sont ajoutés directement au milieu de suspension sous un volume de 20µl apportant la concentration finale voulue (1 mg ou 0.5mg/ml de suspension). Les doses sont exprimées en mg de plante sèche par ml de suspension. Les dosages sont effectués après 30 minutes d'incubation.

### 1.5 Déterminations biochimiques

La lipoperoxydation est exprimée en terme de malonaldéhyde (MAD), déterminée par la méthode à l'acide 2-thiobarbiturique (Bernheim et coll., 1948: "The reaction between thiobarbituric acid and the oxidation products of certain lipids", J. Biol. Chem. 174, 257-264). 1 ml de suspension hépatocytaire est mélangé à 2 ml d'acide trichloroacétique à 10%. Après 10 minutes de repos, les tubes sont centrifugés. 1 ml de surnageant est ajouté à 1 ml d'acide thiobarbiturique à 1%. Les tubes sont alors placés au bain-marie à 100°C pendant 10 minutes puis refroidis avant lecture à 535 nm. Les résultats sont donnés en nMoles/10⁶ cellules.

Les fuites enzymatiques de LDH et d'ASAT sont déterminées par une méthode de cinétique enzymatique à l'aide de kits Boehringer, les résultats sont exprimés en mUl/10⁶ cellules. Les lectures sont faites au spectrophotomètre (Uvikon 810, Kontron), dans l'UV à 340 nm et à 25°C.

### III) Préparation des végétaux

Les drogues (plantes) sèches sont grossièrement contusées puis jetées dans de l'eau distillée bouillante à raison de 60 g de plante sèche pour 600 ml d'eau. La préparation est alors placée à l'étuve à 37°C pendant 4 heures. Les extraits ainsi obtenus sont filtrés sur Büchner, puis concentrés sous pression réduite à une température de 40°C. Une lyophilisation (lyophilisateur CD 52-1, Heto, Danemark) permet leur conservation dans de bonnes conditions. Le rendement exprimé en pourcentage représente le rapport de la quantité de lyophilisat obtenu sur la quantité, en poids sec, de matière végétale utilisée. Toutes les doses mentionnées dans les tests de criblage sont exprimées en mg de plantes sèches.

### 1 Identifications chimiques

Les diverses procédures expérimentales utilisées au cours de l'identification chimique sont celles décrites par Weniger (1984: "La médecine populaire dans le plateaux central d'Haïti", Doctorat de Sciences de l'Université de Metz, Metz).

Pour chaque plante, on a recherché:
- les alcaloïdes,
- les composés flavoniques,
- les saponisides,
- les tannins,
- les dérivés stéroïdiques ou terpéniques.

### 2 Recherche de dérivés stéroïdiques ou terpéniques

On laisse macérer 1g de drogue dans 20 ml d'éther éthylique dans un erhlenmeyer bouché pendant 24 heures. Quelques gouttes de la macération sont évaporées sur un verte de montre et le résidu est dissout dans 2 gouttes d'anhydride acétique. Une coloration allant du vert au mauve après ajout d'une goutte d'H₂SO₄, permet de conclure à la présence probable de dérivés stéroïdiques et/ou terpéniques.

### RESULTATS

Parmi les 26 plantes testées, le *Rosmarinus officinalis* donne les premiers résultats suivants:

### Test d'inhibition du radical DPPH :

Testé dans les conditions précédemment décrites, l'extrait aqueux de *Rosmarinus officinalis* provoque une décoloration de 84% pour une solution de 0.25g/l et de 65% pour une solution de 0.125g/l. Devant ces résultats, Rosmarinus officinalis est clasée en type II (% de décoloration > 50%).

### Test sur hépatocytes isolés:

L'expérimentation est menée selon le protocole décrit précédemment. *Rosmarinus officinalis* présente un effet protecteur à l'encontre de la lipoperoxydation et des phénomènes nécrotiques engendrés par le tert-butyl hydroperoxyde. *Rosmarinus officinalis* (0,5 et 1 mg de plante sèche/ml de suspension hépatocytaire) s'oppose de manière tout à fait significative au relargage enzymatique et à la production de malonaldéhyde, confirmant sur ce modèle son intérêt dans le traitement des affections hépatiques d'origine toxique. L'extrait aqueux de jeunes pousses de *Rosmarinus officinalis* est présenté dans les tests suivants comme produit de référence positif.

### B) ETUDE DE ROSMARINUS OFFICINALIS

### I Etude d'un ajout retardé de Rosmarinus officinalis

Après avoir vérifié que R. officinalis n'influait pas sur les méthodes de mesure utilisées et avant d'aborder la phase de fractionnement, on a cherché à écarter la possibilité d'une éventuelle interaction extracellulaire entre le toxique et l'extrait végétal, à l'aide d'un ajout retardé de l'extrait, 10 minutes après l'intoxication par le tBH. Cette période étant jugée suffisante pour mettre en place les différents facteurs de la nécrose cellulaire. Les résultats obtenus lors de telles expérimentations montrent que l'effet protecteur apparait malgré l'ajout retardé; les phénomènes protecteurs sont donc bien de nature intracellulaire.

### II Influence du solvant d'extraction

Au cours de la phase de criblage, les extractions ont été réalisées en utilisant le solvant le plus souvent retrouvé dans les préparations traditionnelles, l'eau. Avant d'aborder la phase de fractionnement de Rosmarinus officinalis, une série d'extraction différentielle au soxhlet est réalisée à l'aide de solvant de polarité croissante selon la séquence chloroforme, éthyl acétate, éthanol, eau afin de rechercher le solvant d'extraction le plus performant. L'ensemble des travaux de fractionnement est réalisé sur des jeunes pousses (juste après l'éclosion des bourgeons) de romarin. A la réception des plantes, 24 heures plus tard, les jeunes pousses (parties terminales de 2 à 3 cm, vert tendre) sont séparées du reste de la partie aérienne et congelées à -20°C.

### II-1 Soxhlet 1

### II-1 1 Mode opératoire

Un premier soxhlet (Soxhlet I) est réalisé avec 80 g de jeunes pousses de romarin, décongelées et finement contusées avant d'être placées dans la cartouche du soxhlet. Chaque lixiviation est réalisée avec 600 ml de solvant jusqu'à ce que celui-ci ressorte totalement incolore de la cartouche. Les extraits obtenus sont alors concentrés sous vide puis congelés en coque afin d'être lyophilisés. Les quantités de lyophilisats obtenues sont les suivantes :
1.73 g pour l'extraction par le chloroforme
0.24 g pour l'extraction par l'acétate d'éthyle
2.36 g pour l'extraction par l'éthanol
1.94 g pour l'extraction aqueuse
   (80 g de plante fraîche donnent 30.08 g de plante sèche)

Lors du retour à la température ambiante, l'extrait chloroformique se partage en une phase inférieure, liquide, et une phase supérieure, solide et cireuse. Cette dernière provenant probablement de la cuticule des jeunes pousses est séparée par filtration, séchée à l'air et conservée à part. La partie liquide est, après filtration, traitée comme les autres extraits par lyophilisation.

### II-1 2 Contrôle pharmacologique

Les résultats expérimentaux obtenus par les quatre lyophilisats sur hépatocytes isolés après intoxication au *tert*-butyl hydroperoxyde (1,5mM) montrent que l'intoxication est franche tant pour la production de malonaldéhyde qu'en ce qui concerne le relargage enzymatique. Deux extraits, celui à l'éthyl acétate et celui à l'éthanol, ne présentent aucune action pharmacologique sur ce modèle. L'extrait aqueux diminue la fuite enzymatique mais contrairement à un extrait aqueux obtenu par infusion macération reste sans effet sur la formation de MAD. L'extrait chloroformique inhibe totalement la fuite de lactico-déshydrogénase et réduit de manière très significative la production de MAD. L'extrait chloroformique à la dose de 1 mg de plante sèche par ml de suspension d'hépatocytes présente une activité supérieure à tous les extraits aqueux testés au cours du criblage préliminaire.

### II-2 Soxhlet II

### II-2 1 Mode opératoire

Devant ces résultats une deuxième extraction différentielle par soxhlet (Soxhlet II) est réalisée à partir de 120g de jeunes pousses, décongelée, de Rosmarinus officinalis, en utilisant succesivement deux solvants d'extraction, le chloroforme puis l'eau. Le mode de préparation des lyophilisats est identique à celui précédemment décrit. Les quantités de lyophilisats obtenues sont les suivantes :
3.91 g pour l'extrait chloroformique
7.23 g pour l'extrait aqueux

### II-2 2 Contrôle pharmacologique

Les résultats des expérimentations réalisées sur ces nouveaux extraits (non représentés), confirment les travaux précédents. L'extrait chloroformique testé à la dose de 1 mg de plante sèche par ml de suspension, révèle une très forte activité alors que l'extrait correspondant au soxhlet aqueux reste sans effet sur les paramètres étudiés.

### II-3 Caractérisations chromatographiques :

Des profils chromatographiques en couche mince sont alors réalisés sur les différents lyophilisats obtenus (Soxhlet I et Soxhlet II) en vue de la recherche de composés réducteurs, de flavonoïdes et d'acides phénols; ces substances étant susceptibles de porter l'activité selon certains auteurs. Les triterpènes, composés extraits par les solvants faiblement polaires et éventuellement impliqués, eux aussi, dans les phénomènes hépatoprotecteurs (Hikino et coll., 1984: "Antihepatotoxic actions of papyriogenins and papyriosides, triterpenoids of Tetrapanax papyriferum leaves, J. Ethnopharmacol. 12, 231-235) sont également recherchés.

Pour les besoins de l'invention, ne seront détaillées ci-après que les recherches de triterpènes.

### II-3 1 Recherche de triterpènes sur Soxhlet I et Soxhlet II

La recherche de triterpènes est effectuée sur plaque de silice Kieselgel G60 sans fluorescence (20x20; Merck). Le système solvant est constitué d'un mélange benzène/acide acétique dans les proportions 99/1 (v/v). La migration est stoppée lorsque le solvant arrive approximativement à 3 cm du bord supérieur de la plaque. Après séchage, on pulvérise une solution chloroformique de trichlorure d'antimoine à 20 % (m/v). Les plaques sont placées à l'étuve à 120°C pendant 15' puis observées en lumière ultraviolette à 366 nm.

L'extrait obtenu à partir de l'infusion macération de jeunes pousses de romarin et les extraits obtenus par lixiviation aqueuse réalisée lors des deux extractions différentielles par soxhlet ne présentent aucun spot. Les divers extraits chloroformiques donnent le plus grand nombre de taches; celles ci sont d'ailleurs identiquement retrouvées dans ces extraits, en particulier la partie cireuse et le filtrat donnent des profils chromatographiques similaires. Les extraits à l'acétate d'éthyle et à l'éthanol sont également identiques entre eux.

### II-4 Soxhlet III

### II-4 1 Mode opératoire

Devant ces résultats, une troisième extraction par soxhlet est réalisée (Soxhlet III) utilisant cette fois une séquence chloroforme puis méthanol. 100 g de plante fraîche décongelée sont utilisés, le mode de préparation des jeunes pousses de R.officinalis restant identique à ceux précédement décrits. Les quantités de lyophilisats obtenues sont les suivants:
4.2 g pour l'extrait chloroformique
5.6 g pour l'extrait méthanolique

### II-4 2 Contrôle pharmacologique

Les extraits obtenus sont testés sur le modèle in vitro. A nouveau l'extrait chloroformique montre une activité extrêmement intéressante, s'opposant presque complètement à l'action du *tert*-butyl hydroperoxyde. L'extrait méthanolique étant, quant à lui, peu actif sur le relargage enzymatique et la production de MAD.

### II-5 Caractérisation chromatographique

### I-5 1 Recherche de triterpènes

Le protocole employé est celui précédemment décrit. Les extraits chloroformiques issus des deux extractions ont des profils chromatographiques identiques (non représentés ici). L'extraction méthanolique fait apparaître 2 spots en bas de la plaque et 1 spot dans la partie supérieure; le même profil est obtenu avec les fractions à l'éthyl acétate et à l'éthanol.

### II-6 Conclusions et vérifications

L'ensemble de ces résultats fait clairement apparaître :
* la supériorité des extraits chloroformiques par rapport à tous les autres extraits testés, tant sur la formation de malonaldéhyde consécutive à la peroxydation lipidique que sur le relargage enzymatique signant la nécrose cellulaire.
** l'absence apparente, sur les données de la chromatographie sur couche mince, de flavonoïdes dans les extraits chloroformiques et leur présence, dans les extraits sans effet pharmacologique. Cela donne à penser qu'ils n'interviennent pas en tant que support d'activité dans le modèle étudié. L'activité pharmacologique devrait donc en toute logique être porté par des composés révélés par le trichlorure d'antimoine c'est à dire des composés de nature terpènique, stéroïdienne ou caroténoïdienne.

Ces conclusions suscitent deux remarques
* Certains auteurs avaient suspecté que l'activité pharmacologique du romarin reposait sur la présence de certains acides phénols comme l'acide caféique, l'acide chlorogénique et surtout l'acide rosmarinique. Ces substances insolubles ou très peu solubles dans le chloroforme sont entrainées par des solvant tels que l'éthanol, le méthanol ou l'eau.

L'absence d'activité pharmacologique des extraits correspondants a amené à tester ces sustances pures sur le modèle d'hépatocytes isolés, intoxiqués au tert- butyl hydroperoxyde.

Ces composés sont solubilisés dans le DMSO et apportés sous un volume de 20 µl à la suspension hépatocytaire (DMSO= 0.5% v/v) de sorte que leur concentration finale soit de 100 µM (env. 15µg/ml). L'acide rosmarinique représente 1 à 3% de romarin (poid sec) donc 1 mg de plante séche/ml correspond à un poids compris entre 10 et 30 µg d'acide rosmarinique /ml de suspension.

Ni l'acide caféique, ni l'acide chlorogénique ne protégent la cellule hépatique intoxiquée par le *tert*-butyl hydroperoxide. L'acide rosmarinique freine la formation de malonaldéhyde mais reste sans effet sur le relargage enzymatique. Ces substances ne semblent donc pas être responsables de l'activité protectrice apportée par un extrait obtenu par infusion macération.
** Si l'on considère que des substances de nature terpèniques ou stéroïdiennes sont à l'origine de l'activité pharmacologique, il convient alors de vérifier deux hypothéses
   ----> le passage de ce type de substance dans l'infusion macération, extrait pharmacologiquement actif car capable d' inhiber la production de MAD et le relargage enzymatique provoqué par le *tert*-butyl hydroperoxyde.
   ----> l'existence d'une différence d'activité entre l'infusion macération et l'extraction aqueuse au soxhlet après passage de chloroforme qui aura eu pour effet d'entrainer ces composés.

### ** Recherche de triterpènes dans les extraits aqueux et hydroalcooliques

Mode opératoire : 700 mg de lyophilisat obtenu par infusion macération de jeunes pousses de romarin, à 37°C pendant 4 heures, sont solubilisés dans de l'eau distillée. Une extraction liquide-liquide par du chloroforme est alors réalisée. L'agitation est maintenue pendant une heure. Après un temps de repos, les deux phases sont séparées, concentrées sous vide à 40°C puis lyophilisées. La fraction aqueuse ramène 563.4 mg; la fraction chloroformique ramène 92 mg.

Une extraction au chloroforme (100 ml) a également été réalisée sur 250 ml de teinture mère préparée de manière industrielle (TM Dolisos lot n°10769, TA=62°9, 21 jours de macération). Deux fractions sont séparées, après agitation pendant une heure et mise au repos, puis concentrées sous vide.

Caractérisation chromatographique : La recherche des triterpènes se fait sur chaque fraction obtenue selon la méthodologie précédemment décrite. Le solvant utilisé pour la migration est le chloroforme, le révélateur le trichlorure d'antimoine. Le dépôt correspondant au soxhlet chloroformique est 20 fois moins concentré que les autres dépôts.

L'extrait chloroformique obtenu par soxhlet (Soxhlet II) donne le nombre de spots le plus élevé (non représenté). Les fractions aqueuses de l'infusion macération et de la teinture mère ne donnent aucun spot au trichlorure d' antimoine. Par contre pour les fractions chloroformiques, on met en évidence quatre taches en bas de plaque pour la fraction issus de l'infusion macération et un nombre de spots plus élevé pour la fraction issue de la teinture mère. L'infusion macération à 37°C, pendant 4 h, est donc un mode d'extraction qui permet le transfert de composés relativement peu polaires, facilement extraits par le chloroforme lors d'une séparation liquide-liquide secondaire. La présence de ces composés, susceptibles d'être porteurs de l'activité hépatoprotectrice pourrait par conséquent expliquer l'activité pharmacologique de l'extrait réalisé par infusion macération et leur concentration, inférieure à celle retrouvée dans un extrait chloroformique, le niveau de protection plus faible. La présence d'un nombre de taches plus important pour la fraction chloroformique de la teinture mère étant probablement en relation avec le temps d'extraction plus long (21 jours).

### ** Comparaison de l'activité d'extraits aqueux selon le type d'extraction.

Le protocole utilisé lors de l'expérimentation in vitro est celui précédemment décrit. Tous les extraits sont testés à la dose de 1mg plante sèche par ml de suspension. La concentration finale de DMSO est de 0.5% v/v.

Les résultats montrent, de nouveau, une activité supérieure de l'extrait chloroformique par rapport aux extraits aqueux, tant dans l'inhibition des processus peroxydatifs que de l'atteinte nécrotique. L'effet de l'extrait aqueux issu de l'infusion macération est supérieur à celui de l'extrait obtenu par soxhlet.

Ces résultats accréditent la thèse selon laquelle l'activité hépatotrope du romarin semble être le fait de composés peu polaires.

Au terme de ces études, les extraits chloroformiques de Rosmarinus officinalis apparaissent, sans équivoque, comme les plus performants en terme d'hépatoprotection sur le modèle expérimental retenu. Un fractionnement par chromatographie préparative sur couche mince est alors effectué sur ces extraits dans le but d'obtenir la purification la plus complète possible des principes

ctifs. Il est réalisé à partir de l'extrait chloroformique Soxhlet II de jeunes pousses de Rosemarinus officinalis.

### III Fractionnement de l'extrait chloroformique par chromatographie préparative

### ** Méthodologie générale

La veille des fractionnements, des plaques de verre (20x20), propres et dégraisées, sont recouvertes de 0.5 mm d'un mélange de gel de silice G60 Merck (45g) et d'eau distillée (90g), à l'aide d'un applicateur de Stahl. Les plaques sont séchées à l'air puis à l'étuve à 100°C pendant 2 heures. Les plaques sont passées à l'étuve pendant une heure, à 100°C, juste avant utilisation.

Le choix du solvant du migration est guidé à chaque nouvelle chromatographie préparative par les migrations précédents; le premier solvant utilisé est le chloroforme. Après migration du solvant sur 15 cm, les plaques sont retirées puis séchées à l'air. Différentes zones de migration sont alors individualisées afin de réaliser un contrôle pharmacologique permettant d'orienter les fractionnements ultérieurs.

### III-1 Fractionnement primaire

### III-1 1 Mode opératoire

50 mg de lyophilisat de l'extrait chloroformique Soxhlet II sont solubilisés dans 1 ml de chloroforme puis déposés (0.1 ml/cm) sur la plaque de silice a l'aide d'un applicateur de Firmenicht. Le solvant utilisé est le chloroforme. Après migration, les plaques sont retirées de la cuve puis séchées à l'air. Une des plaques est utilisée pour la recherche des composés terpèniques à l'aide d'une solution chloroformique de trichlorure d'antimoine à 20%. L'autre sert à la poursuite du fractionnement. En lumière naturelle d'une part et en UV, a 366 nm, après pulvérisation du révélateur et passage à l'étuve à 120°C d'autre part, on obtient six zones (non représentées):
- une zone dépôt, correspondant à l'endroit où le dépôt a été effectué,
- 4 zones de migration,
- une zone témoin située en dessous de la zone dépôt est également individualisée.

Chaque zone est délicatement grattée du support en verte et la silice est placée dans des erlenmeyers de 25 ml. Deux lavages successifs, avec 20 ml de chloroforme sous agitation magnétique, sont réalisés. Après chaque lavage une centrifugation est effectuée pour séparer le surnageant de la silice; un troisième lavage est effectué avec 20 ml de méthanol, plus polaire, et après centrifugation, le surnageant méthanolique est ajouté aux surnageants chloroformiques dèjà obtenus. Chaque fraction est alors concentrée sous vide à 40°C jusqu'à un volume de 1 à 2 ml puis versée dans un tube à hémolyse en verre où elle est conservée après évaporation à sec à température ambiante sous un courant d'azote.

### III-1 2 Caractérisation chromatographique

Un contrôle chromatographique est alors effectué et comparé à celui de l'extrait chloroformique Soxhlet II de départ. Deux systèmes de solvants sont utilisés, le chloroforme d'une part et un solvant, d'élution plus importante, constitué par un mélange chloroforme - méthanol dans les proportions de 95/5. En lumière UV, après pulvérisation du trichlorure d'antimoine et passage à l'étuve, les différents spots de l'extrait chloroformique de départ sont retrouvés dans les zones 1 à 4. La zone de dépôt et la zone témoin ne présentent aucun spot avec ce système révélateur.

### III-1 3 Contrôle pharmacologique

Les différentes fractions obtenues sont ensuite testées sur hépatocytes isolés, intoxiqués au tBH (1,5 mM), après avoir été solubilisées dans le DMSO de telle sorte que la concentration finale par ml de suspension hépatocytaire corresponde à la quantité de fraction contenue dans 1 mg de plante sèche. Le nombre de fractions individualisées par chromatographie a volontairement été restreint pour pouvoir apprécier l'action pharmacologique des différentes fractions simultanément et ainsi comparer leurs activités sur des populations cellulaires en tous points homogènes. Chaque expérimentation est effectuée au moins deux fois. L'effet protecteur sur la nécrose cellulaire est apprécié en terme de LDH relarguée, l'activité antilipoperoxydante par la diminution de la MAD formée.

On peut résumer aussi l'activité des zones issues de ce premier fractionnement. La zone témoin sans effet pharmacologique, n'est pas représentée sur les graphes. L'extrait chloroformique de départ (Soxhlet II) utilisé à la dose de !mg de plante sèche par ml de suspension sert de témoin. La fuite enzymatique est bien freinée par les fractions dépôt, 1, 2 et 3. La zone 4, la plus éluée, est inactive. La sensibilité de la MAD est plus grande, pour toutes les expérimentations les zones ayant le moins migrées (dépôt et zone 1) présentent l'activité la plus importante. Celle-ci est d'ailleurs comparable à celle de l'extrait chloroformique témoin. A la vue de ces résultats le fractionnement se poursuit à partir de la zone dépôt et de la zone 1.

### III-2 Fractionnement de la zone dépôt

### III-2 1 Mode opératoire

La fraction dépôt est solubilisée dans 1 ml de chloroforme puis déposée en bande à l'aide d'un applicateur manuel sur des plaques préparées selon le protocole précédemment décrit. Le solvant utilisé est un mélange chloroformeméthanol dans les proportions 90/10 v/v afin d'augmenter la force d'élution par rapport à la séparation précédente. La migration est stoppée lorsque le front de solvant a parcouru 15 cm, la plaque est alors séchée et observée en lumière naturelle. Comme précédemment cinq zones sont individualisées, certaines zones regroupant plusieurs bandes (non représentées). Ces zones numérotées de D1 à D5, de bas en haut de la plaque, sont grattées de leur support en verre, placées dans des erlenmeyers et subissent deux lavages au chloroforme suivis d'un lavage au méthanol selon les conditions précédemment décrites. Concentrées sous vide, les fractions sont ensuite placées dans des tubes à hémolyse et évaporées à sec.

### III-2 2 Contrôle pharmacologique

On effectue les contrôles expérimentaux sur hépatocytes isolés, intoxiqués au tert butyl hydroperoxyde. L'extrait de départ (zone Dépôt) est testé simultanément pour servir de référence. L'activité sur les phénomènes peroxydatifs et les phénomènes nécrotiques est retrouvée pour le témoin dépôt. Par contre les différentes fractions ne présentent aucune activité pharmacologique. La tentative de purification de cette fraction est arrêtée à ce stade.

### III-3 Fractionnement de la zone 1

### III-3 1 Mode opératoire

La fraction zone 1 est solubilisée dans 1 ml de chloroforme avant d'être déposée selon le protocole précédemment décrit. Le solvant utilisé est une solution chloroforme - méthanol (90/10). Comme pour la zone dépôt, cinq zones sont individualisées (non représentées), certaines zones regroupant plusieurs bandes. Ces zones sont numérotées de 1.1 à 1.5 de bas en haut de la plaque, elles sont grattées, lavées deux fois au chloroforme puis une fois au méthanol. Les fractions sont concentrées sous vide, puis évaporées à sec dans des tubes à hémolyse.

### III-3 2 Contrôle pharmacologique

Les test in vitro réalisés sur ces fractions montrent que les zones 1.3 et 1.4 conservent une activité importante. Ces fractions sont à leur tour, purifiées par chromatographie préparative en couche mince.

### III-4 Fractionnement de la zone 1.3

### III-4 1 Mode opératoire

Après solubilisation de la fraction 1.3, le protocole utilisé est identique a ceux précédemment décrits. Le solvant de migration contient 85% de chloroforme et 15% de méthanol afin d'augmenter le pouvoir éluant. Après migration et séchage, 9 zones sont individualisées, numérotées de 1.3.1. à 1.3.9 de bas en haut de la plaque (non représentées). Chaque zone est grattée puis lavée au chloroforme et au méthanol, les surnageants concentrés sous vide et évaporés à sec selon les conditions déjà citées.

### III-4 2 Contrôle pharmacologique

L'activité pharmacologique tant sur la MAD que sur la LDH (non représentée) est retrouvée sans discussion pour la fraction 1.3 de départ servant de référence. Par contre la purification a dilué l'activité de telle sorte qu'aucune fraction ne présente un effet pharmacologique notable. La tentative de purification pour ces fractions est naturellement arrêtée à ce stade.

### III-5 Fractionnement de la zone 1.4

### III-5 1 Mode opératoire

La fraction 1.4 est solubilisée dans 1 ml de chloroforme et déposée sur une plaque selon la procédure habituelle. Du fait de la position de la zone 1.4 lors de la séparation précédente, un solvant moins éluant, constitué d'un mélange de chloroforme et d'acétate d'éthyle (85/15 v/v), est utilisé. Lorsque le solvant a parcouru 15 cm, la plaque est retirée et séchée. Après séchage on constate que la moitié inférieure de la zone de migration est de couleur ocre et qu'il n'y apparaît aucune bande clairement individualisée. On effectue alors une seconde migration jusqu'à la première bande visible lors de la première migration (non représentée) à l'aide d'un mélange chloroforme/méthanol (90/10 v/v).

Après séchage, la plaque est divisée en six zones numérotées de 1.4.1 à 1.4.6 de bas en haut. On applique alors à chacune de ces zones la procédure de lavage et d'élimination des solvants déjà indiquée.

### III-5 2 Contrôle pharmacologique

Afin d'être testée expérimentalement, chacune des zones est reprise par du DMSO de telle sorte que la quantité finale / ml de suspension hépatocytaire corresponde à 1 mg de plante séche. La fraction 1.4 de départ sert de référence, elle inhibe les phénomènes peroxydatifs et nécrotiques induits par le tert-butyl hydroperoxyde de manière très significative (non représentée). La fraction 1.4.2 reste très active, surtout sur le relargage enzymatique. La fraction 1.4.3 présente une activité moins importante, les quatre autres fractions sont inefficaces. Devant la faible quantité de fraction 1.4.2, la purification est arrêtée à ce stade et il est décidé de tenter d' identifier les composants de cette fraction.

### IV Identification de la fraction 1.4.2.

### IV-1 Chromatographie en couche mince sur la fraction 1.4.2

### IV-1 1 Identification de la fraction 1.4.2 par le trichlorure d'antimoine

Le dépôt de la fraction 1.4.2 est réalisé sur une plaque recouverte de silice Kieselgel G60 Merck. L'acide ursolique, l'acide oléanolique, l'α amyrine et la β amyrine sont utilisés comme témoins. Ces substances, déjà isolées dans les parties aériennes de R. officinalis, sont susceptibles d'être extraites par les systèmes de solvants utilisés. Le solvant de migration est un mélange chloroforme-méthanol dans des proportions de 90/10. Le révélateur est le trichlorure d'antimoine à 20% dans le chloroforme. La lecture se fait à 366 nm, après passage à l'étuve à 120°C pendant 10'. L'acide ursolique, l'acide oléanolique, l'α amyrine donnent des spots jaunâtres (non représentée). Le spot de la β amyrine, situé au même niveau que celui de l'α amyrine est plus orangé. La fraction 1.4.2 donne un spot, situé au niveau de ceux des acides oléanolique et ursolique mais de coloration plus ocre.

### IV-1 2 Identification de la fraction 1.4.2 par le sulfate de cérium

Le support, les témoins et le solvant de migration sont identique à ceux décrit au paragraphe précédent. Le révélateur est un mélange sulfate de cérium - acide sulfurique (d=1,84) et recherche d'éventuels dérivés du tocophérol. On retrouve les mêmes spots de couleur orangée pour l'acide ursolique, oléanolique, l' α amyrine et la fraction 1.4.2, la β amyrine présente une coloration gris-vert (non représentée) Aucun spot supplémentaire n'est mis en évidence par cette technique par rapport à ceux visualisés après pulvérisation de trichlorure d'antimoine.

### IV-1 3 Identification de la fraction 1.4.2 par l'aldéhyde anisique

Les dépôts sont effectués sur une plaque recouverte de silice Kieselgel G60 Merck. L'α et la β amyrine, les acides ursolique et oléanolique et l'extrait chloroformique Soxhlet II sont utilisés comme témoins. Après migration, on pulvérise le révélateur suivant :

| | |
|---|---|
| méthanol | 85 |
| acide acétique glacial | 10 |
| acide sulfurique à 95% | 5 |
| aldéhyde anisique | 0.5 |

Deux systèmes de solvant sont utilisés pour les migrations
* un système chloroforme-méthanol (90/10) déjà utilisé auparavant permet de retrouver les spots précédemment décrits (non représentés), l'α et la β amyrine proche du front de solvant de couleur ocre, un peu plus bas l'acide oléanolique (spot violet) et ursolique (spot gris). L'extrait chloroformique Soxhlet II donne naturellement le nombre de tache le plus élevé. La fraction 1.4.2 donne un seul spot visible de couleur gris-violet et de Rf voisin de celui des acides oléanolique et ursolique.
* un système toluène-acétate d'éthyle (95/5) est utilisé pour tenter de visualiser la présence éventuelle de composés plus apolaires dans la fraction 1.4.2. Les témoins restent les mêmes que précédemment. Le profil obtenu n'est pas représenté. Un grand nombre de spots, correspondant aux composés les plus apolaires de l'extrait chloroformique Soxhlet II, sont effectivement retrouvés. Les autres témoins se situent dans la moitié inférieure de la plaque, tout comme le spot de la fraction 1.4.2, ce solvant de migration ne permet donc pas de visualiser de nouvelles taches pour cette fraction.

A la vue de tous ces résultats il apparaît que la fraction 1.4.2, pharmacologiquement active, semble relativement bien purifiée. Un seul spot apparaît en CCM après pulvérisation de révélateurs susceptibles de mettre en évidence des composés retrouvés dans l'extrait chloroformique de départ, notamment des composés de nature terpèniques ou stéroïdes. Le seul spot mis en évidence se situe dans une zone voisine de celle des spots des acides ursolique et oléanolique, que l'on sait être présents dans le romarin et dont certains auteurs avaient retenu la possible activité pharmacologique. Pour vérifier la présence de ces composés dans la fraction 1.4.2 et pour pallier aux possibilités d' investigation rapidement limitées des techniques de chromatographie sur couche mince sur des échantillons de faible quantité, une identification de la fraction 1.4.2 par chromatographie en phase gazeuse couplée à une spectrométrie de masse est entreprise. Parallélement, une purification sur plaque est effectuée après méthylation de la très faible quantité restante de la fraction 1.4.2.

### V-2 Chromatographie en phase gazeuse couplée à la spectrométrie de masse

Afin d'augmenter la volatilité des composants de la fraction 1.4.2, celle ci est dérivée par sylilation. L'addition de groupe triméthylsylile permet d'augmenter la volatilité des composés avec une bonne stabilité chimique et thermique. Les agents de sylilation utilisés ont une réactivité relativement large, pouvant notamment toucher des groupements R-OH, Ar-OH ou R-COOH.

Une chromatographie en phase gazeuse est alors effectuée. La température de l'injecteur est de 280°C, celle de la colonne de 220°C; après une phase isothermique de 5'. l'augmentation de la température est de 5 °C par minute.

L'α et la β amyrine, l'acide oléanolique, l'acide ursolique sont utilisés comme témoins. La purification de cette fraction est satisfaisante, moins de vingt composés étant retrouvés. Parmi ceux ci, on peut distinger deux groupes principaux.
- un groupe de trois composés apparaissant entre 8 et 9 minutes (groupe 1).
- un groupe de quatre composés apparaissant plus tardivement entre 22 et 24 minutes après l'injection (groupe 2).

La comparaison avec les chromatogrammes des témoins et l'étude des spectres de masse permet d'établir :
- l'absence d'α et de β amyrine dans la fraction 1.4.2.
- la présence d'acide oléanolique dans la fraction 1.4.2.
- la présence d'acide ursolique dans la fraction 1.4.2.
- le groupe apparaissant le plus tardivement renferme deux autres composés; dont l'un très minoritaire n'est pas identifiéet l'autre donne un spectre de masse qui peut faire penser à une structure trés proche de celle de l'acide ursolique.
- enfin le groupe 1 comprend trois composés non identifiés.

### IV-3 Purification de la fraction 1.4.2 après méthylation

Une partie de la fraction 1.4.2 est alors méthylée au diazométhane, puis séparée par une nouvelle chromatographie préparative sur couche mince (gel de silice G60) à l'aide d'un solvant de migration chloroforme/éther de pétrole (75/25). Quatre fractions sont invidualisées, les fractions supérieures (2,3,4) susceptibles de contenir des substances terpèniques déjà identifié par la CPG/SM précédente sont écartées. Seule la fraction 1, correspondant à la zone de dépôt, est à nouveau séparée sur gel de silice à l'aide d'un système éluant chloroforme / acétate d'éthyle (90/20 v/v). Deux spots sont alors obtenus (1a et 1b). La fraction la, obtenue en quantité infime, possède un spectre de masse qui n'apporte pas d'élément d'orientation particulier, par contre le spectre de masse réalisé en impact électronique à partir de la zone 1b montre un ion moléculaire à 342 pouvant correspondre à la lutéoline tétraméthylée (non représenté). Des ions de fragmentation caractéristiques de ce composé étant également retrouvés.

### IV-4 Conclusions

Au terme de cette tentative de fractionnement, les seuls composés clairement identifiés comme étant présents dans la fraction 1.4.2 et par voie de conséquence porteurs potentiels de l'activité pharmacologique, sont des triterpènes.

Une série d'expérimentations a donc été mise en oeuvre sur hépatocytes isolés, intoxiqués au tert butyl hydroperoxyde, pour apprécier l'impact des triterpènes identifiés sur les mécanismes hépatotoxiques.

### V Activité pharmacologique des composés identifies de la fraction 1.4.2.

### V-1 Recherche de l'activité pharmacologique des composés terpèniques

Au cours d'une première expérimentation, l'acide ursolique, l'acide oléanolique et l'α amyrine sont testés à la dose de 100 µM. La quercétine, utilisée à la même dose, sert de substance de référence.

Aucune activité pharmacologique n'est retrouvée pour l'acide oléanolique et l'acide ursolique sur le modèle utilisé, ces composés ne sont donc pas responsable de l'activité de la fraction 1.4.2 issus d'un extrait chloroformique de jeunes pousses de R. officinalis dans le modèle expérimental utilisé.

Quant à la lutéoline, présente dans la fraction 1.4.2 pourrait donc, au terme de cette étude, être retenue comme possible porteur de l'activité hépatoprotectrice des jeunes pousses de R. officinalis. Il convenait donc d'apprécier la quantité de lutéoline présente dans l'extrait chloroformique et l'extrait 1.4.2 afin de vérifier si celle-ci était suffisante pour entrainer l'activité pharmacologique.

Or, il a été déterminé qu'elle est présente à des doses trop faibles pour expliquer, à elle seule, l'activité pharmacologique.

Une nouvelle tentative de purification de l'extrait chloroformique sohxlet II de jeunes pousses de R.officinalis est donc entreprise afin d'approcher de manière plus précise les composés responsables de l'activité pharmacologique. Cet essai utilise une technique de séparation différente de la précédente et est réalisée sur chromatotron.

### VI Fractionnement de l'extrait chloroformique de R.officinalis par chromatotron

Le chromatotron (Harrison Research 7924T) permet une chromatographie séparative sur couche mince, accélérée par effet centrifuge. La solution à séparer est appliquée près du centre d'un disque recouvert d'une couche d'absorbant et tournant à grande vitesse; l'élution par les différents solvants utilisés se faisant du centre vers la périphérie. La migration des différentes bandes peut être suivie en illuminant le disque par une lumière UV (254 nm). Les plaques sont réalisées la veille de chaque manipulation. 45 grammes de Kieselgel 60 PF 254 Merck sont mélangés à 90 grammes d'eau distillée. Le mélange est réparti (1mm d'épaisseur), à l'aide d'un applicateur, sur un disque de verre, propre et dégraissé avec soin, de 20 cm de diamètre. Les disques sont séchés à l'air puis à l'étuve à 70°C pendant 5 heures. Le jour de la manipulation les disques sont réactivés par un passage à l'étuve pendant une heure.

### VI-1 Chromatotron I

### VI-1 1 Mode opératoire

La première séparation est réalisée à partir de 100 mg de lyophilisat de l'extrait chloroformique Sohxlet II. Le chloroforme est le seul solvant d'élution utilisé. Le fractionnement est arrêté lorsque l'éluant est incolore et que les bandes n'évoluent plus. Les différentes fractions réalisées au cours du temps sont regroupées en quatre lots (A, B, C, D) correspondant à un volume d'éluant de 75 ml chacune. Les quatre fractions sont concentrées sous vide à 40°C, jusqu'à l'obtension d'un résidu sec.

### VI-1 2 Contrôle pharmacologique

Pour être testées pharmacologiquement les quatre fractions sont reprises par du DMSO de telle sorte que la concentration finale corresponde à 1 mg de plante sèche par ml de suspension d'hépatocytes. Les résultats (non représentés) montrent le comportement similaire sur la formation de malonaldéhyde et les libérations de lactate déshydrogénase et d'aspartate amino-transférase de ces quatre fractions. L'activité pharmacologique s'accroit au fur et à mesure du temps d'élution, la fraction obtenue la plus tardivement étant la plus active. Ces résultats font apparaître que les principes actifs de l'extrait chloroformique de jeunes pousses de romarin bien qu'extraient en totalité par le Soxhlet chloroformique (les extraits de polarité supérieure étant inactifs) montrent une affinité relativement peu importante pour les solvants très apolaires.

On a confirmé ce point en effectuant une extraction liquide - liquide utilisant un mélange hexane - chloroforme (1/1 v/v) à partir d'un lyophilisat d'extrait chloroformique Soxhlet II. Les fractions obtenues après une heure d'agitation sont évaporées à sec puis reprises par le DMSO pour être testées à une concentration correspondant à 1mg de plante sèche / ml de suspension hépatocytaire. Les résultats expérimentaux montrent clairement que la fraction hexanique contenant les composés les plus apolaires n'est pas pharmacologiquement active, le reste de l'extrait chloroformique étant très actif. Des profits chromatographiques réalisés sur la partie hexanique n'ont mis en évidence ni flavonoïdes par le réactif de NEU (diphénylboryloxyéthylamine), ni composés terpèniques par le trichlorure d'antimoine.

### VI-2 Chromatotron II

### VI-2 1 Mode opératoire

Une deuxième séparation est réalisée à partir de 100 mg de lyophilisat de l'extrait chloroformique Soxhlet II. Trois solvants sont utilisés successivement, d'abord le méthanol puis l'éthyl acétate enfin le chloroforme jusqu'à nettoyage de la plaque. Les différentes fractions réalisées au cours du temps sont regroupées de façon à obtenir deux fractions méthanoliques (F1, F2) de 75 ml chacune, et deux fractions à l'éthyl acétate (F3, F4) de 75 ml chacune; toutes les fractions chloroformiques étant regroupées (F5).

### VI-2 2 Caractérisations chromatographiques

### * recherche de composés terpèniques et stèroïdiens

Le protocole utilisé a été décrit précédemment, le solvant utilisé est un mélange benzène - acide acétique (99/1). Le révélateur est le trichlorure d'antimoine. Les témoins utilisés sont les extraits chloroformiques Soxhlet II et Soxhlet III et l'extrait méthanolique Soxhlet III (non représentés). La première fraction méthanolique (F1) issue de l'extrait chloroformique Soxhlet II présente quatre des cinq spots de l'extrait de départ, le cinquième spot situé en milieu de plaque n'apparaissant dans aucune des fractions. F1 présente deux spots non retrouvés sur l'extrait méthanolique Soxhlet III qui est sans action pharmacologique.

### VI-2 3 Contrôle pharmacologique

Les différentes fractions (F1 à F5) sont reprises par le DMSO de sorte que la concentration finale corresponde à 1 mg de plante sèche/ml de suspension. L'activité pharmacologique de ces fractions sur les phénomènes peroxydatifs et le relargage enzymatique est représentée. L'intoxication est franche pour les trois paramètres considérés. La première fraction méthanolique entraine une hépatoprotection manifeste, les autres extraits sont totalement inefficaces.

### VI-3 Chromatotron III

### VI-3 1 Mode opératoire

En fonction des résultats précédents, une troisième procédure de séparation est effectuée à partir de 100 mg de lyophilisat d'un extrait chloroformique (Soxhlet II) de jeunes pousses de R. officinalis. Dans un premier temps quatre fractions sont isolées selon la procédure suivante :
* fraction 1 < --- passage de 75 ml de chloroforme
* fraction 2 < --- passage de 75 ml supplémentaire de chloroforme
* fraction 3 <--- passage de 75 ml de méthanol
* fraction 4 <--- passage de 75 ml d'un mélange méthanol-acide acétique (95/5 v/v)

La fraction 3, après concentration au rotovapor, est reprise sur chromatotron selon la séquence suivante :
* fraction 3.1 <--- passage de 75 ml d'un mélange CHCl3 + CH3COOH (95/5 v/v)
* fraction 3.2 <--- passage de 75 ml suplémentaire d'un mélange CHCl3 + CH3COOH (95/5 v/v)
* fraction 3.3 <--- passage de 75 ml de méthanol

Après concentration, la fraction 3.3 est elle même repassée sur chromatotron
* fraction 3.3.1 <--- passage de 75 ml de CHCl3
* fraction 3.3.2 <--- passage suplémentaire de 75 ml de CHCl3
* fraction 3.3.1 <--- passage de 75ml de CH3COOH

### VI-3 2 Caractérisation chromatographique

La recherche de composés de nature stéroïdes et terpèniques est réalisée, sur les différentes fractions obtenues, par CCM (gel de silice G60 Merck) en utilisant le chloroforme comme solvant de migration.

### VI-3 3 Contrôle pharmacologique

Les différentes fractions sont reprises par le DMSO afin d'étudier leur activité antilipoperoxydante et antinécrotique. Les doses testées sont équivalentes à 1 mg de plantes sèches / ml de suspension hépatocytaire. Les résultats montrent clairement que la fraction 3.2 est la plus efficace tant sur les phénomènes peroxydatifs que sur les fuites enzymatiques signant la nécrose cellulaire.

### VI-3-4 Identification de la fraction 3.2

La fraction 3.2 est analysée par chromatographie en phase gazeuse après silylation. La température de l'injecteur est de 180°C, le programme de température du four débute à 240°C, reste stable pendant 1 minute puis augmente de 10°C par minute jusqu'à 280°C.

Cinq composés majoritaires sont retrouvés (non représentés). Leurs masses varient de 279 à 515 (non représentés). En comparant les spectres de masse de la fraction 3.2 avec ceux des composés issus de la fraction 1.4.2., on constate que l'on retrouve un et un seul composé commun à ces deux fractions. Le composé A de masse égale à 504 possède en effet un spectre de masse superposable à celui présenté (non représenté). On peut donc conclure que les deux procédures de fractionnement retenues, qui utilisent des méthodologies différentes (chromatotron et C.C.M. préparative) permettent d'isoler un même composé dans les fractions pharmacologiques les plus actives. Ce composé peut donc être considéré comme porteur principal de l'activité pharmacologique (antilipoperoxydante et antihépatotoxique) de Rosmarinus officinalis.

### VI-3-5 Identification du composé porteur de l'activité pharmacologique.

En raison des procédures utilisées au cours du fractionnement et des spectres de masse obtenus, a été émise l'hypothèse que le composé porteur de l'activité hépatotrope pourrait appartenir au groupe des lactones triterpèniques présents dans le romarin et dont les composés principaux ont été primitivement isolés de Salvia officinalis. Ces composés, dont les chefs de file sont le carnosol et le rosmanol présentent d'ailleurs une remarquable activité antioxydante dans divers tests, non biologiques, utilisés en vue de la recherche d'antioxydants alimentaires (test de l'oxydation des "tranches de lard") (Inatani et coll., 1982: "Structure of the new antioxydative phenolic diterpene isolated from Rosmary", Agri. Biol. Chem., 46, 1661 et suivantes). Le composé présentant un m/z à 562 de la fraction 1.4.2. peut correspondre au rosmanol. Le principe actif retenu de m/z à 504 doit correspondre à un méthyl-rosmanol, la présence du groupement méthyle empêchant la sylilation du groupement hydroxyle correspondant (562-73 + 15 = 504).

Cette hypothèse a été confirmée en testant l'activité pharmacologique du rosmanol.

### C) CONTROLE DE L'ACTIVITE PHARMACEUTIQUE DU ROSMANOL

L'activité pharmacologique du rosmanol est confirmée sur le modèle d'hépatocytes isolés intoxiqués au tert-butyl hydroperoxyde. 25 mM de rosmanol inhibent totalement la formation de malonaldéhyde et réduisent très significativement les phénomènes nécrotiques.

Les résultats montrant l'activié pharmacologique du rosmanol sont rassemblés dans le tableau ci-après:

| | MAD | LDH | ASAT |
|---|---|---|---|
| DMSO | 0.31 ± 0.06 | 422 ± 72 | 29 ± 13 |
| tBH 1,5 mM | 6.82 ± 0.80 | 2428 ± 250 | 116 ± 15 |
| tBH + rosmanol 100 mM | 0.25 ± 0.05 | 268 ± 28 | 20 ± 1 |
| tBH + rosmanol 50 mM | 0.32 ± 0.06 | 350 ± 88 | 22 ± 2 |
| tBH + rosmanol 25 mM | 0.35 ± 0.08 | 434 ± 73 | 31 ± 2 |

## Revendications

1. Composition pharmaceutique caractérisée en ce qu'elle contient, comme substance active l'un au moins des composés de formule I: 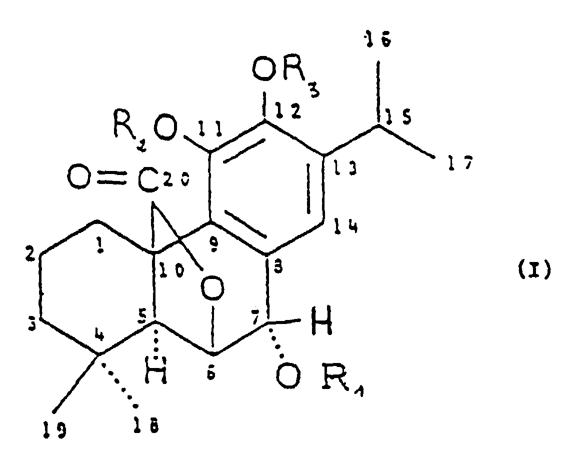 dans laquelle R₁, R₂, R₃ identiques ou différents représentent H, CH₃ ou C₂H₅, éventuellement en association avec un sel pharmaceutiquement acceptable.

2. Composition pharmaceutique selon la revendication 1, caractérisée en ce qu'elle contient, comme substance active le composé de formule II:

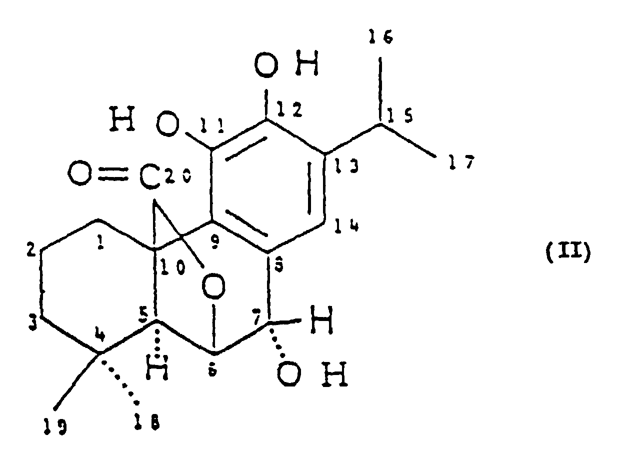

3. Composition pharmaceutique selon la revendication 1, caractérisée en ce qu'elle contient, comme substance active, le composé de formule III, IV ou V:

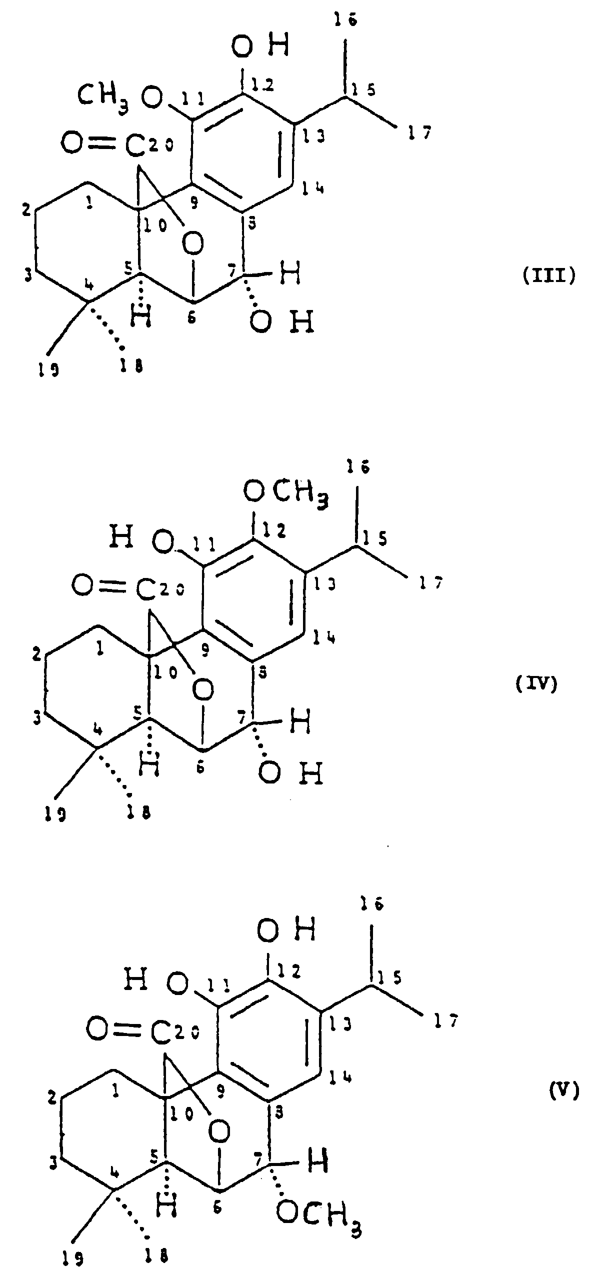

4. Composition pharmaceutique selon la revendication 1, caractérisée en ce qu'elle contient, comme substance active, le composé de formule VI, VII ou VIII:

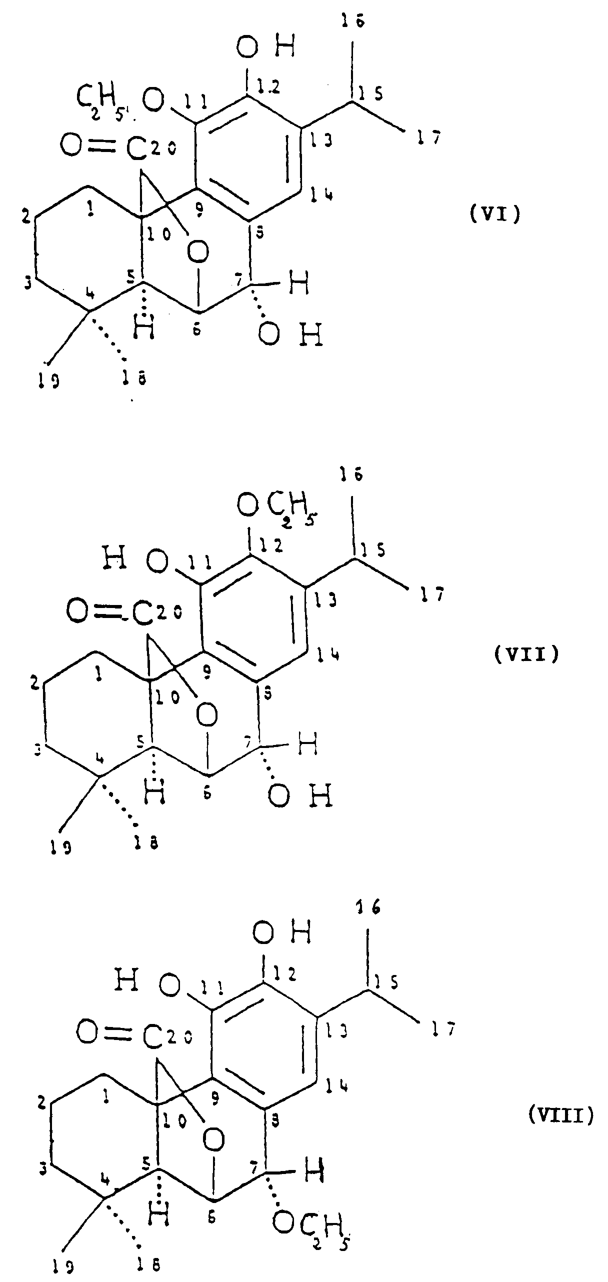

5. Compositions pharmaceutiques ayant des propriétés antiradicalaires et/ou antilipoperoxydantes et/ou hépatotropes, et contenant comme substance active l'un au moins des composés de formule I selon la revendication 1, ou l'un au moins des composés de formule II, III, IV, V, VI, VII ou VIII selon l'une quelconque des revendications 2, 3, ou 4.

6. Composition pharmaceutique selon l'une quelconque des revendications 1 à 3, caractérisée en ce qu'elle contient d'environ 0,1 mg à environ 100 mg de substance active par kg de poids corporel, et notamment d'environ 1 mg/kg à environ 10 mg/kg de substance active.

7. Composition pharmaceutique selon la revendication 6, caractérisée en ce qu'elle contient d'environ 0,1 mg à environ 100 mg de substance active par kg de poids corporel, et notamment d'environ 1 mg/kg à environ 10 mg/kg de substance active constituée de l'un au moins des composés de formule I et notamment de formule II, III, IV, V, VI, VII ou VIII et particulièrement de formule II, V, ou VIII.

8. Composition pharmaceutique selon l'une quelconque des revendications 1 à 7, caractérisée en ce qu'elle se présente sous une forme appropriée à l'administration par voie orale, parentérale rectale ou topique.

9. Composition pharmaceutique selon l'une quelconque des revendications 1 à 8, caractérisée en ce que la substance active est issue de *Rosmarinus officinalis.*

10. Utilisation de l'un au moins des composés de formule I, 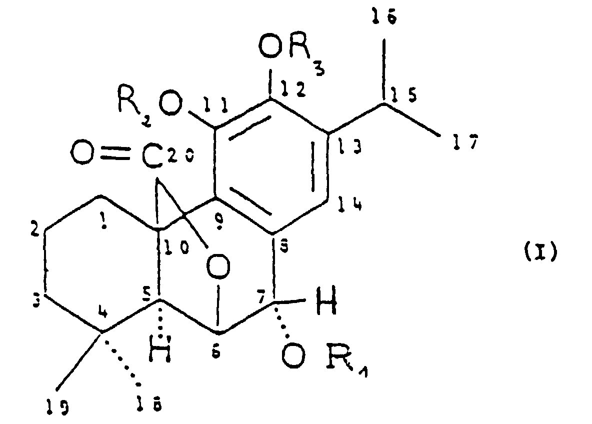 dans laquelle R₁, R₂, R₃ identiques ou différents représentent H, CH₃ ou C₂H₅. dans laquelle R₁, R₂, R₃ identiques ou différents représentent H, CH₃ ou C₂H₅. pour la préparation d'un médicament destiné au traitement de pathologies liées à des mécanismes de formation de radicaux libres, ou au traitement de pathologies liées à un mécanisme de lipoperoxydation, ou au traitement ou à la protection des cellules hépatiques

11. Utilisation du composé de formule II, 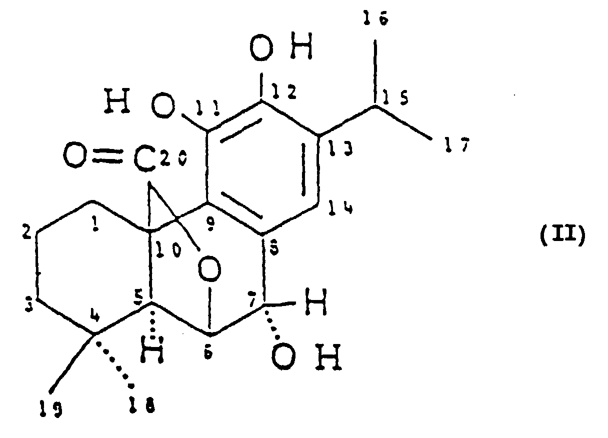 pour la préparation d'un médicament destiné au traitement de pathologies liées à des mécanismes de formation de radicaux libres, ou au traitement de pathologies liées à un mécanisme de lipoperoxydation, ou au traitement ou à la protection des cellules hépatiques.

12. Utilisation du composé de formule V, 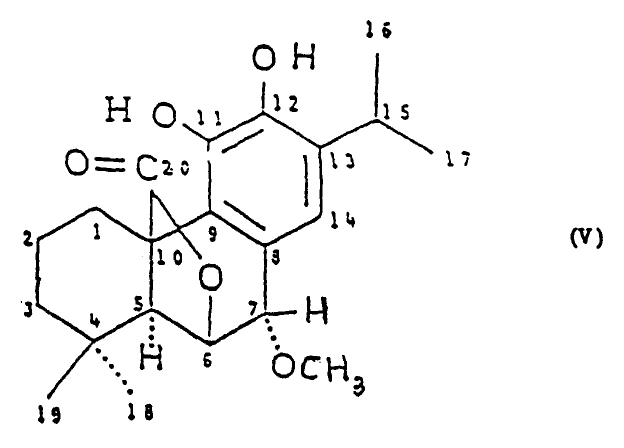 pour la préparation d'un médicament destiné au traitement de pathologies liées à des mécanismes de formation de radicaux libres, ou au traitement de pathologies liées à un mécanisme de lipoperoxydation, ou au traitement ou à la protection des cellules hépatiques.

13. Utilisation du composé de formule VIII, 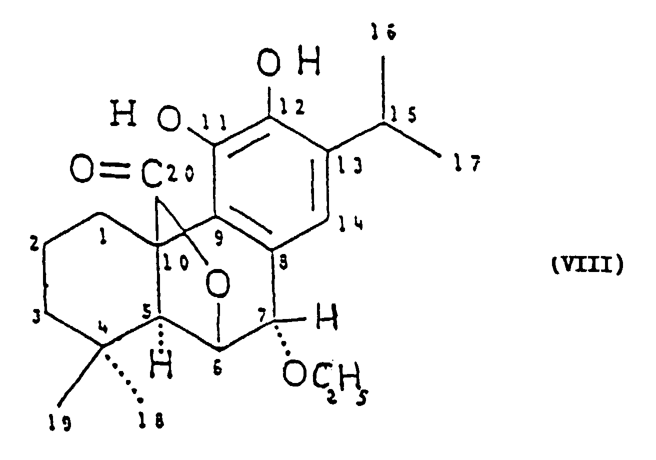 pour la préparation d'un médicament destiné au traitement de pathologies liées à des mécanismes de formation de radicaux libres, ou au traitement de pathologies liées à un mécanisme de lipoperoxydation, ou au traitement ou à la protection des cellules hépatiques.

## Claims

1. A pharmaceutical composition characterised in that it contains, as active substance, at least one of the compounds corresponding to formula I: 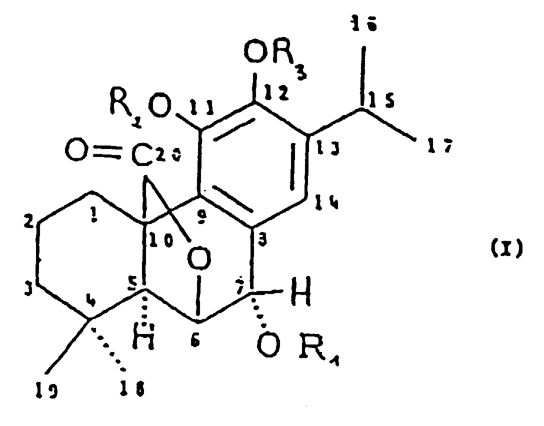 wherein R₁, R₂, R₃, which are identical or different, represent H, CH₃ or C₂H₅, possibly in association with a pharmaceutically acceptable salt.

2. A pharmaceutical composition according to claim 1, characterised in that it contains, as active substance, the compound corresponding to formula II:

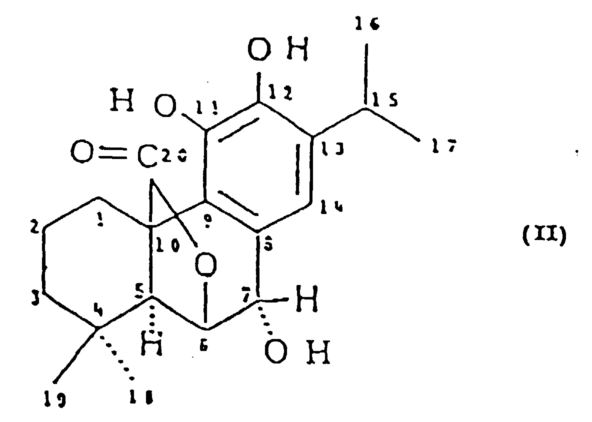

3. A pharmaceutical composition according to claim 1, characterised in that it contains, as active substance, the compound corresponding to formula III, IV or V:

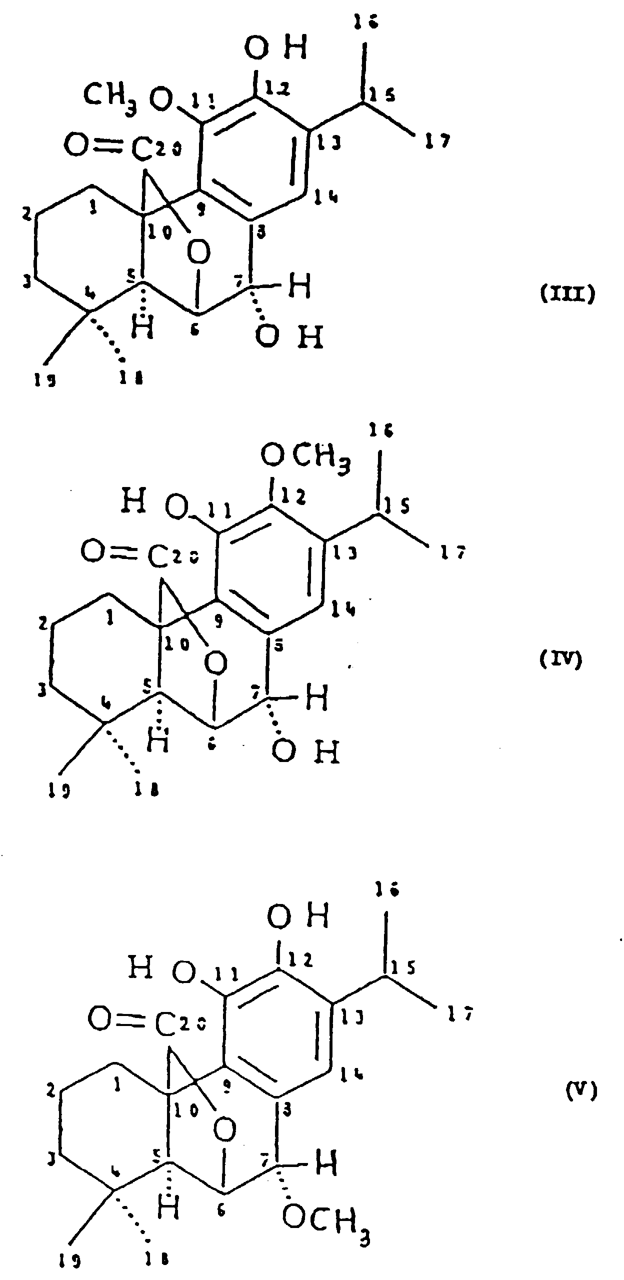

4. A pharmaceutical composition according to claim 1, characterised in that it contains, as active substance, the compound corresponding to formula VI, VII or VIII:

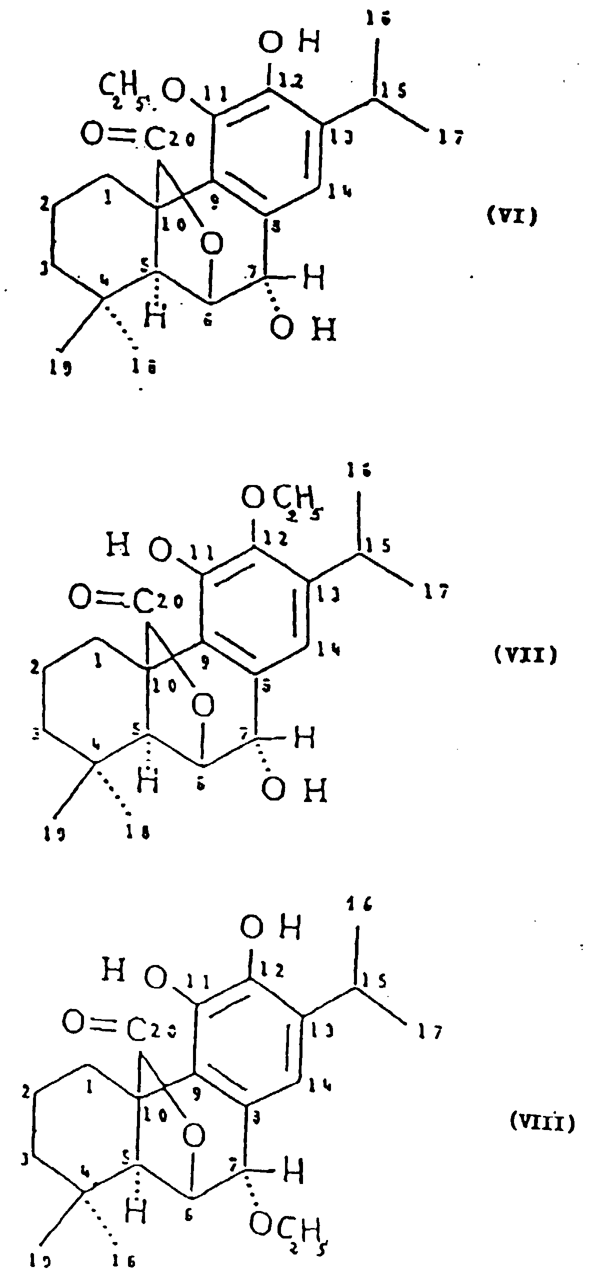

5. Pharmaceutical compositions having antiradical and/or antilipoperoxidative and/or hepatotropic properties and containing, as active substance, at least one of the compounds corresponding to formula I according to claim 1, or at least one of the compounds corresponding to formula II, III, IV, V, VI, VII or VIII according to any one of claims 2, 3, or 4.

6. A pharmaceutical composition according to any one of claims 1 to 3, characterised in that it contains about 0.1 mg to about 100 mg of active substance, per kg of body weight, and in particular about 1 mg to about 10 mg of active substance.

7. A pharmaceutical composition according to claim 6, characterised in that it contains about 0.1 mg to about 100 mg of active substance, per kg of body weight, and in particular about 1 mg/kg to about 10 mg/kg of active substance, per kg of body weight, composed of at least one of the compounds corresponding to formula I and in particular to formula II, III, IV, V, VI, VII or VIII and particularly to formula II, V or VIII.

8. A pharmaceutical composition according to any one of claims 1 to 7, characterised in that it is present in a form suitable for oral, parenteral, rectal or topical administration.

9. A pharmaceutical composition according to any one of claims 1 to 8, characterised in that the active substance is derived from *Rosmarinus officinalis.*

10. The use of at least one of the compounds corresponding to formula I, 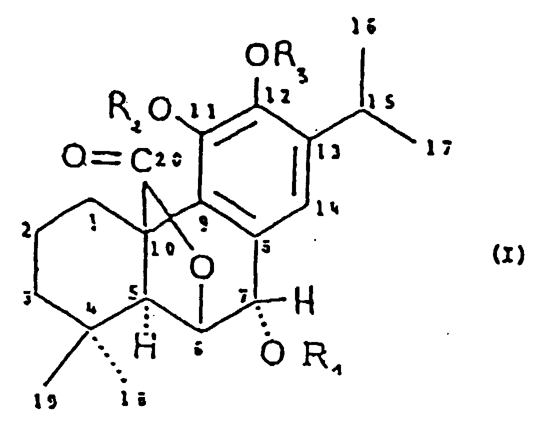 wherein R₁, R₂, R₃, which are identical or different, represent H, CH₃ or C₂H₅, for the preparation of a medicinal product intended for the treatment of pathologies associated with mechanisms of free radical formation, or for the treatment of pathologies associated with a lipid peroxidation mechanism, or for the treatment or protection of hepatic cells.

11. The use of the compound corresponding to formula II 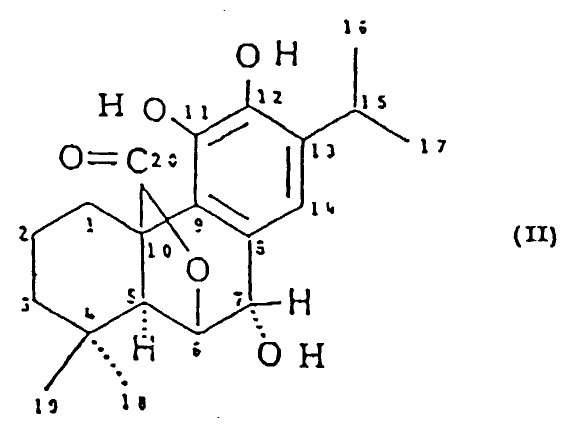 for the preparation of a medicinal product intended for the teatment of pathologies associated with mechanisms of free radical formation, or for the treatment of pathologies associated with a lipid peroxidation mechanism, or for the treatment or protection of hepatic cells.

12. The use of the compound corresponding to formula V 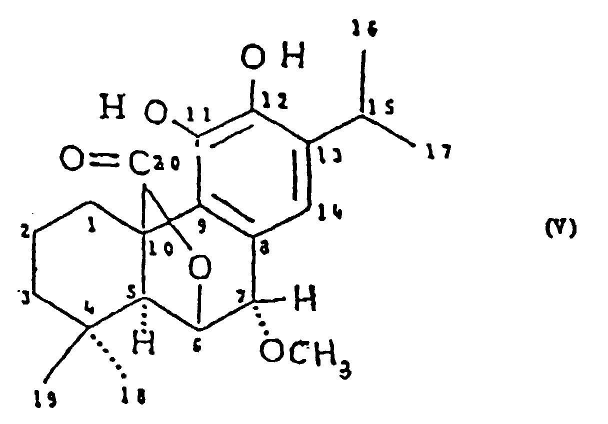 for the preparation of a medicinal product intended for the treatment of pathologies associate with mechanisms of free radical formation, or for the treatment of pathologies associated with a lipid peroxidation mechanism, or for the treatment or protection of hepatic cells.

13. The use of the compound corresponding to formula VIII 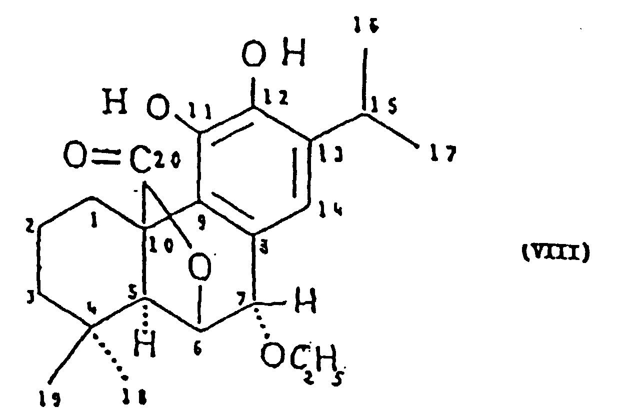 for the preparation of a medicinal product intended for the treatment of pathologies associated with mechanisms of free radical formation, or for the treatment of pathologies associated with a lipid peroxidation mechanism, or for the treatment or protection of hepatic cells.

## Patentansprüche

1. Pharmazeutische Zusammensetzung, dadurch gekennzeichnet, daß sie als aktiven Besandteil mindestens eine der Verbindungen der Formel I 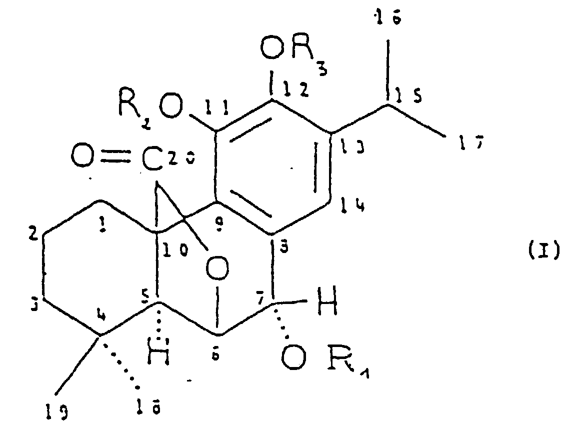 enthalt, worin R₁, R₂, R₃, die gleich oder verschieden sein können, H, CH₃ oder C₂H₅ bedeuten, gegebenenfalls zusammen mit einem pharmazeutisch brauchbaren Salz.

2. Pharmazeutische Zusammensetzung nach Anspruch 1, dadurch gekennzeichnet, daß sie als aktive Substanz die Verbindung der Formel II 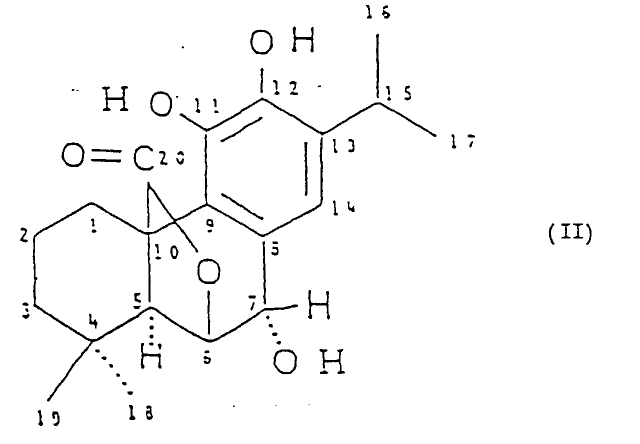 enthält.

3. Pharmazeutische Zusammensetzung nach Anspruch 1, dadurch gekennzeichnet, daß sie als aktive Substanz die Verbindung der Formel III, IV oder V 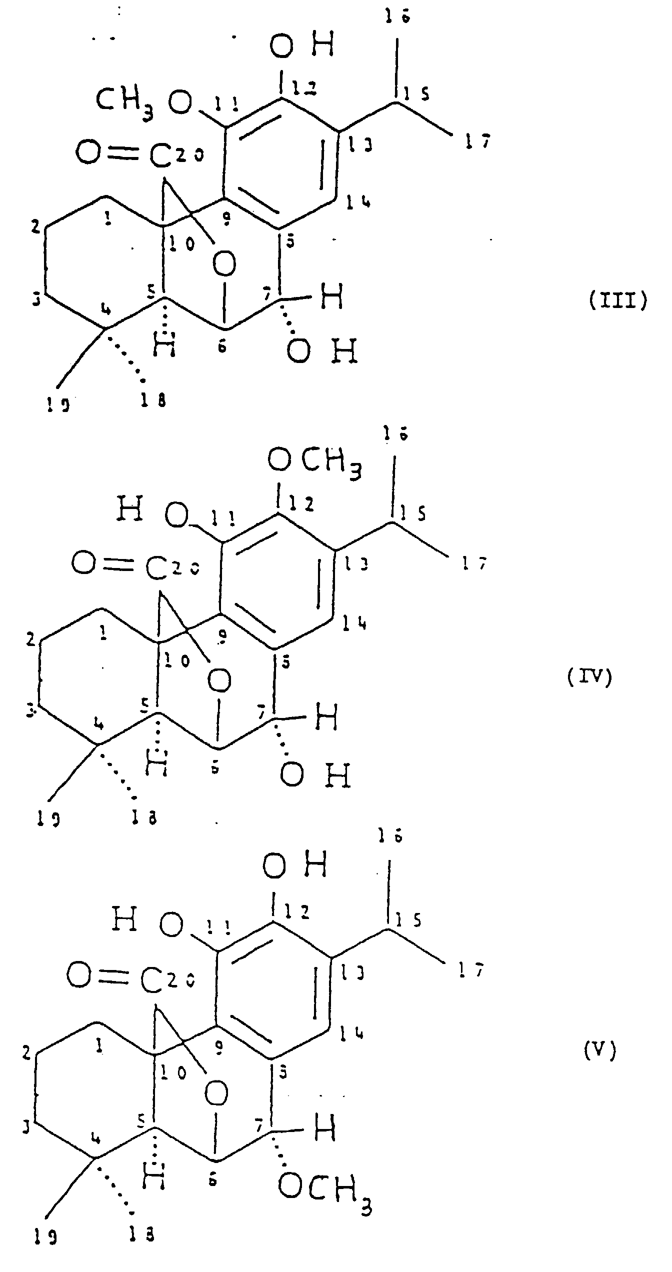 enthält.

4. Pharmazeutische Zusammensetzung nach Anspruch 1, dadurch gekennzeichnet, daß sie als aktive Substanz die Verbindung der Formel VI, VII oder VIII 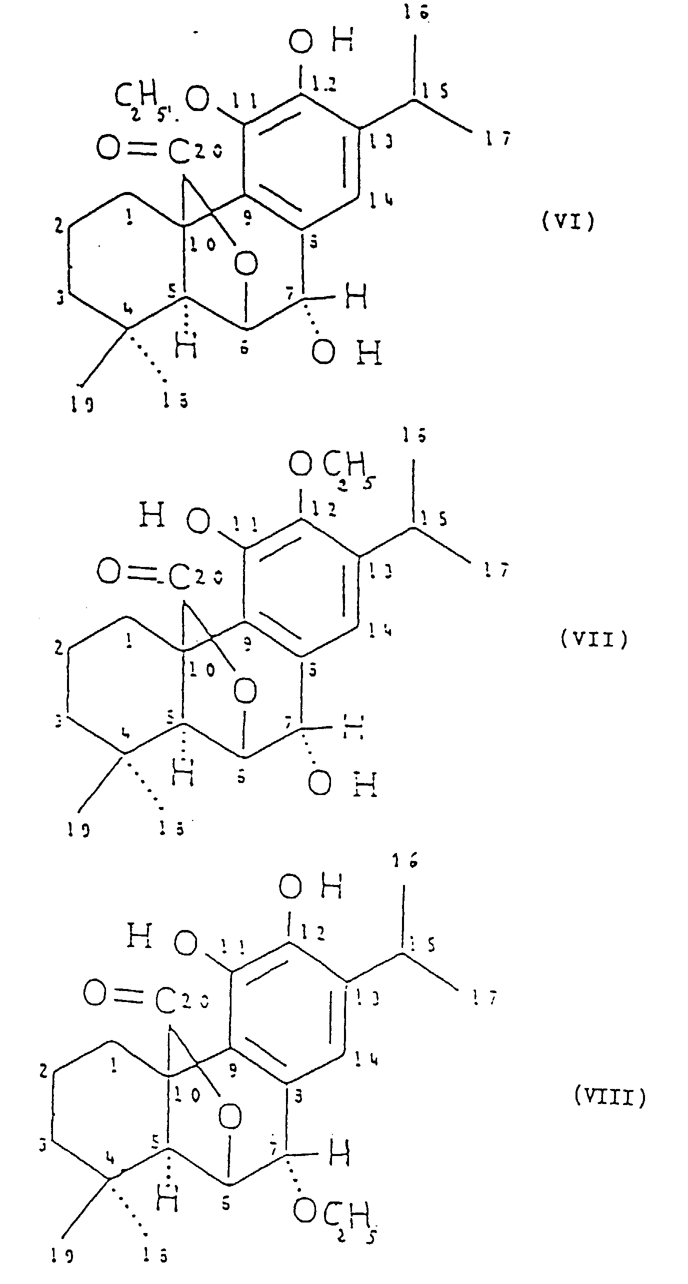 enthält.

5. Pharmazeutische Zusammensetzungen mit antiradikalischen und/oder antilipoperoxydativen und/oder hepatotropen Eigenschaften, enthaltend als wirksame Substanz mindestens eine der Verbindungen der Formel I nach Anspruch 1 oder mindestens eine der Verbindungen der Formel II, III, IV, V, VI, VII oder VIII nach einem der Ansprüche 2, 3 oder 4.

6. Pharmazeutische Zusammensetzung nach einem der Ansprüche 1 bis 3, dadurch gekennzeichnet, daß sie etwa 0,1 mg bis etwa 100 mg aktiver Substanz pro kg Körpergewicht und insbesondere etwa 1 mg/kg bis etwa 10 mg/kg der aktiven Substanz enthält.

7. Pharmazeutische Zusammensetzung nach Anspruch 6, durch gekennzeichnet, daß sie etwa 0,1 mg bis 100 mg aktiver Substanz pro kg Körpergewicht und insbesondere etwa 1 mg/kg bis etwa 10 mg/kg aktiver Substanz enthält, die gebildet wird aus mindestens einer der Verbindungen der Formel I und insbesondere der Formel II, III, IV, V, VI, VII oder VIII und ganz besonders der Formel II, V oder VIII.

8. Pharmazeutische Zusammensetzung nach einem der Ansprüche 1 bis 7, dadurch gekennzeichnet, daß sie in geeigneter Form zur Verabreichung auf oralem, parenteralem, rektalem oder topischem Wege vorliegt.

9. Pharmazeutische Zusammensetzung nach einem der Ansprüche 1 bis 8, dadurch gekennzeichnet, daß die aktive Substanz von Rosmarinus officinalis stammt.

10. Verwendung mindestens einer der Verbindungen der Formel I 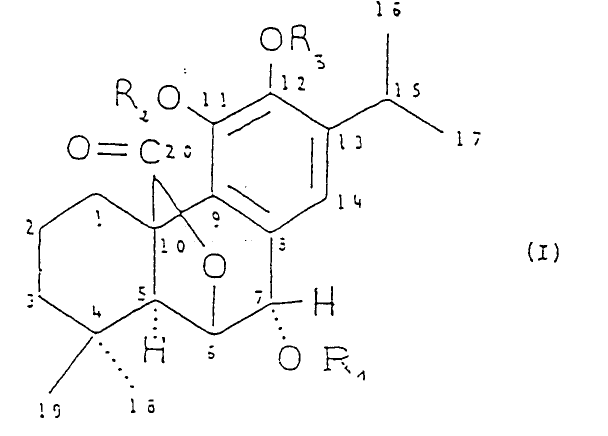 worin R₁, R₂, R₃, die gleich oder verschieden sein können, H, CH₃ oder C₂H₅ sind, zur Herstellung eines Arzneimittels, das zur Behandlung Von Erkrankungen bestimmt ist, die mit Mechanismen zur Bildung freier Radikaler einhergehen oder zur Behandlung Von Erkrankungen, die mit einem Lipoperoxidationsmechanismus einhergehen oder zur Behandlung oder zum Schutz von Leberzellen.

11. Verwendung der Verbindung der Formel II 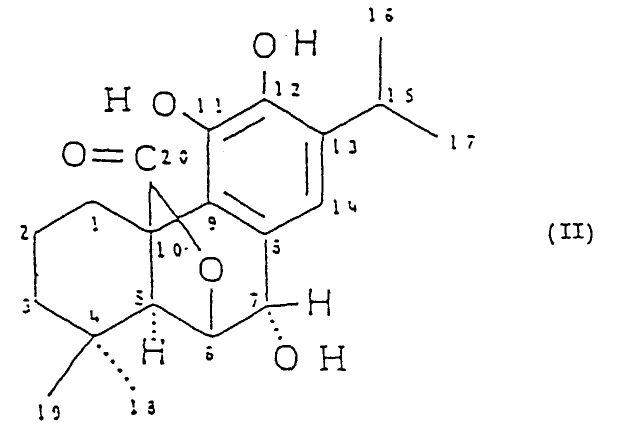 zur Herstellung eines Arzneimittels, das zur Behandlung von Erkrankungen bestimmt ist, die mit Mechanismen zur Bildung freier Radikaler einhergehen oder zur Behandlung von Erkrankungen, die mit einem Lipoeroxidationsmechanismus einhergehen oder zur Behandlung oder zum Schutz von Leberzellen.

12. Verwendung einer Verbindung der Formel V 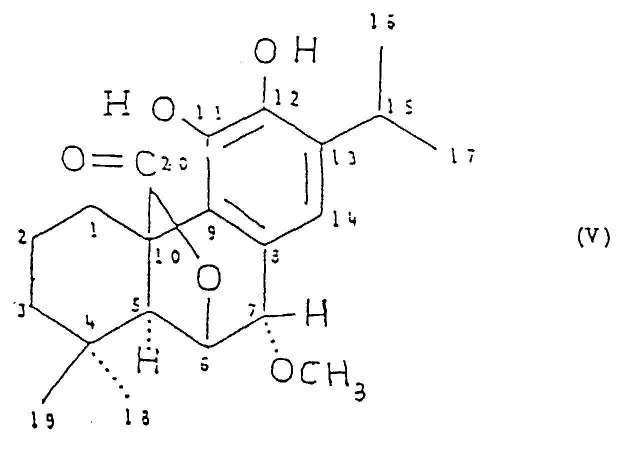 zur Herstellung eines Arzneimittels, das zur Behandlung von Erkrankungen bestimmt ist, die mit Mechanismen zur Bildung freier Radikaler einhergehen oder zur Behandlung von Erkrankungen, die mit einem Lipoperoxidationsmechanisms einhergehen oder zur Behandlung oder zum Schutz von Lebenzellen.

13. Verwendung der Verbindung der Formel VIII 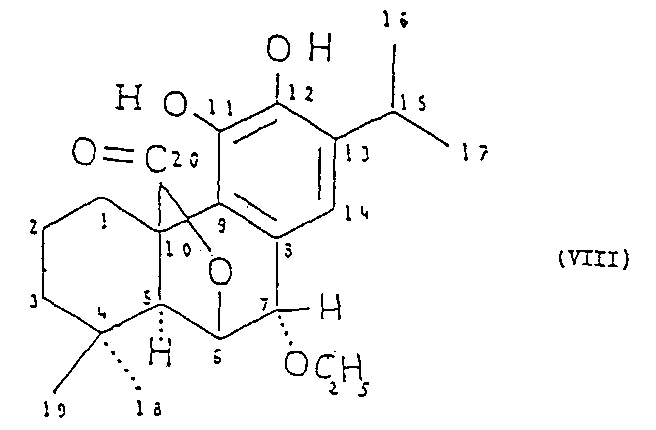 zur Herstellung eines Arzneimittels, das zur Behandlung von Erkrankungen bestimmt ist, die mit Mechanismen zur Bildung freier Radikaler einhergehen oder zur Behandlung von Erkrankungen, die mit einem Lipoperoxidationsmechanismus einhergehen oder zur Behandlung oder zum Schutz von Leberzellen.
